(19) Europäisches Patentamt
European Patent Office
Office européen des brevets

(11) Veröffentlichungsnummer: **0 135 162**
**B1**

(12) **EUROPÄISCHE PATENTSCHRIFT**

(45) Veröffentlichungstag der Patentschrift: **04.04.90**

(51) Int. Cl.⁵: **C 07 C 219/02**

(21) Anmeldenummer: **84109849.4**

(22) Anmeldetag: **17.08.84**

(54) **Weinsäuremonoester von optisch aktiven Alkanolaminen, Verfahren zu ihrer Herstellung und ihre Verwendung.**

(30) Priorität: **19.08.83 DE 3330005**

(43) Veröffentlichungstag der Anmeldung:
**27.03.85 Patentblatt 85/13**

(45) Bekanntmachung des Hinweises auf die Patenterteilung:
**04.04.90 Patentblatt 90/14**

(84) Benannte Vertragsstaaten:
**AT BE CH DE FR GB IT LI LU NL SE**

(56) Entgegenhaltungen:
**HELVETICA CHIMICA ACTA, Band 65, Nr. 1, 1982, Seiten 377-384, Schweizerische Chemische Gesellschaft, CH; V. PRELOG et al.: "Über die Enanatiomerentrennung durch Verteilung zwischen flüssigen Phasen"**

**AUSTRALIAN JOURNAL OF CHEMISTRY, Band 32, Nr. 1, Januar 1979, Seiten 65-70, AU; K.H. BELL: "Kinetic resolution in the reaction of (2R,3R)-2,3-diacetoxy- and (2R,3R)-2,3-dibenzoyloxy-succinic anhydrides with racemic alcohols"**

(73) Patentinhaber: **Lindner, Wolfgang, Dr.**
**St. Veiter-Anger 22**
**A-8046 Graz (AT)**

(72) Erfinder: **Lindner, Wolfgang, Dr.**
**St. Veiter-Anger 22**
**A-8046 Graz (AT)**

(74) Vertreter: **Dipl.-Phys.Dr. Manitz Dipl.-Ing., Dipl.-W.-Ing. Finsterwald Dipl.-Ing. Grämkow Dipl.-Chem.Dr. Heyn Dipl.-Phys. Rotermund Morgan, B.Sc.(Phys.) Robert-Koch-Strasse 1**
**D-8000 München 22 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 135 162 B1

**Beschreibung**

Die Erfindung betrifft Weinsäuremonoester von optisch aktiven, substituierten Alkanolaminen, ein Verfahren zu ihrer Herstellung sowie ihre Verwendung zur Herstellung der optisch reinen Alkanolamine aus den Weinsäuremonoestern.

Einige substituierte Alkanolamine der allgemeinen Formel

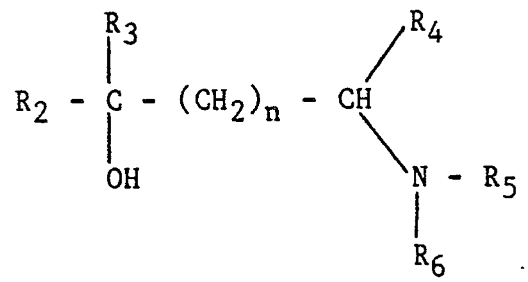

(II)

in der

n null bis vier bedeutet,

$R_2$ Alkyl, Aralkyl, Aryl, Methylenfluorenonoximether, die Gruppe $R_7$-oxyalkyl, in der $R_7$ Aryl oder ein heterocyclischer Rest ist, wobei die Reste jeweils substituiert sein können, oder Wasserstoff bedeutet,

$R_3$ Aryl, Alkyl, Cycloalkyl, wobei die Reste jeweils substituiert sein können, oder Wasserstoff bedeutet,

$R_4$ Alkyl, Aryl, wobei die Reste substituiert sein können, oder Wasserstoff bedeutet,

$R_5$ und $R_6$, die gleich oder verschieden sein können, geradkettiges, verzweigtes oder cyclisches Alkyl, einen 5- oder 6-gliedrigen stickstoffhaltigen Heterocyclus, Aralkyl oder Aminoalkylcarbonyl, wobei die Reste jeweils substituiert sein können, oder Wasserstoff bedeutet,

und in der die Reste $R_2$ und $R_5$ und/oder $R_5$ und $R_6$ ringförmig miteinander verknüpt sein können, sind bekannt.

Insbesondere substituierte Ethanol- und Propanolamine haben als Cardiaca pharmazeutische große Bedeutung.

Es sind verschiedene Verfahren bekannt, um optisch reine substituierte Alkanolamine herzustellen. So kann man

a) entweder über die asymmetrische Synthese zu optisch mehr oder weniger reinen Alkanolaminen gelangen oder aber

b) das racemische Gemisch synthetisieren und anschliessend auf direktem oder indirektem Weg einer Racematrennung unterziehen.

Zur Gruppe a) zählt die Möglichkeit, von einer optisch aktiven Verbindung auszugehen, dei z.B. als optisch reines Naturprodukt vorliegt, z.B. D-Mannitol, das dann oxidativ zum optisch aktiven C3-Bruchstück, der Glycidyl-Gruppe, gespalten wird. Aus diesem werden dann optisch aktive Ethanol- bzw. Propanolamine synthetisiert. (Vgl. DE—OS 28 10 732). Es besteht auch die Möglichkeit, über stereospezifische Reaktionen, z.B. stereospezifische Katalyse, Induktion oder Inversion zu optisch aktiven Verbindungen zu gelangen. Im Gegensatz zum obigen Verfahren führt dieser Weg jedoch kaum zu optischen Ausbeuten der Endprodukte von >98%, wenn man nicht Racemattrennverfahren wie im Verfahren b) auf direktem oder indirektem Weg mitverwendet. (Vgl. D. Valentine und I. W. Scott, Review "Assymmetric Organic Synthesis", Synthesis (1978) 329).

Nach dem Verfahren b) sind bereits Racemattrennungen an Beispielen der als β-Blocker verwendeten Alkanolamine beschrieben. Der Weg der indirekten Racemattrennung besteht darin, daß von racemischen Produkt (z.B. mit freier primärer oder sekundärer Aminogruppe) mit optisch reinen Reaktionspartnern diastereomere Reaktionsprodukte hergestellt, und diese dann bevorzugt chromatographisch in ihre optischen Isomeren aufgetrennt werden. Als optisch aktive Reagenzien werden Arylalkylisocyanate (z.B. S(-)-1-Phenylethylisocycanat) (vgl. W. Dieterle und W. Faigle, J. Chromatogr. 259 (1983) 311), Säurehalogenide von optisch aktiven Säuren (z.B. N-Trifluoracetyl-L-Prolyl-chlorid) (vgl. S. Caccia et al., J. Chromatgr. Science (1978) 543) oder reaktive N-blockierte Aminosäurederivate (z.B. symmetrisches Anhydrid von BOC-L-Leucin) (vgl. J. Hermansson und C. von Bahr, J. Chromatogr. 227 (1982) 113) verwendet. Alle diese Reagenzien haben für präparative Zwecke dan Nachteil eines hohen Preises und führen gewöhnlich zu Produkten mit einem Amidstrukturelement. Die nach diesen Verfahren hergestellten diastereomeren Derivate dienen bevorzugt analaytischen Zwecken und sind auf Normalphase-, z.B. Kieselgel oder Reversed-Phase-, z.B. RP 18-Trennsystemen zu chromatographieren. Auch die Gas-Chromatographie kann in besonderen Fällen eingesetzt werden. Die Selektivitätsfaktoren α, als Maß für die Trennung der diastereomeren Gemische, reichen meist aus, aum eine Basislinientrennung in analytischen Trennsystemen zu erzielen. Eine Hydrolyse der Amidstrukturelemente zu den optisch reinen Alkanolaminen gelingt nur unter verschärften Reaktionsbedingungen, wodurch stets die Gefahr einer Racemisierung besteht.

Eine andere direkte Racemattrennmethode besteht darin, racemische Alkanolamine mit optisch aktiven Säuren unter Bildung von diastereomeren Salzen umzusetzen, die unterschiedliche Kristalleigenschaften (unterschiedliche Lipophilie) aufweisen (Verfahren der stereospezifischen fraktionierten Kristallisation). Hier sind jedoch meist verlustreiche wiederholte Kristallisationsschritte notwendig, um Endprodukte mit hoher optischer Reinheit zu erhalten. Nach der Trennung können aus den Salzen leicht im basischen Milieu die Alkanolamine freigesetzt werden. Als optisch aktive Säuren dienen z.B. Campher-10-sulfonsäure (vgl. P. Newman, Optical Resolution Procedures for Chemical Compounds, Vol. 1, Optical Resolution Information Center, New York, 10471), (R,R)- bzw. (S,S)-O,O-Dibenzoyl- oder O,O-Ditoluoyl-Weinsäure (vgl. T. Leigh, Chem. Ind. London, 36 (1977) oder R. Howe, British Patent 1,069,343 (1967)). Statt der stereospezifischen Kristallisation kann unter bestimmten Voraussetzungen bei diesen Salzen auch die chromatographische Enantiomerentrennung verwendet werden, wobei es sich hierbei nun ebenfalls um eine direkte Racemattrennungsmethode handelt. Die Selektivitätsfaktoren sind jedoch meist zu gering, und eine präparative Anwendung ist nur schwer gangbar.

In Aust. J. Chem., 1979, *32*, Seite 65—70 wird eine als "Kinetic Resolution" bezeichnete Technik beschrieben, bei der verschiedene racemische Alkohole, also Verbindungen mit nur einer funktionellen Gruppe, mit (2R,3R)-2,3-Diacetoxy- und (2R,3R)-2,3-Dibenzoyloxy-bernsteinsäureanhydriden umgesetzt werden. Bei diesem Verfahren wird ein kleiner Teil der großem Überschuß eingesetzten racemischen Alkohole mit einem der genannten Anhydride zum entsprechenden Weinsäuremonoester umgesetzt, wobei die Reaktion des einen Enantiomeren des racemischen Alkohols als Folge sterischer Effekte kinetisch bevorzugt ist. Bei den erhaltenen diastereomeren Weinsäuremonoestern überwiegt das Diastereomere, welches das kinetisch bevorzugt reagierende Alkohol-Enantiomer enthält. Somit erfolgt keine Racemattrennung in die optisch reinen Weinsäuremonoester oder Alkohole, sondern nur eine Verschiebung des Enantiomerenverhältnis des racemischen Alkohols zu einem leichten überschuß des einen Enantiomeren. Im Fall des Oktan-2-ol beträgt die optische Reinheit nach der Hydroylyse des Esters nur 12,1%. Optisch reine Weinsäuremonoester sind in dieser Druckschrift somit weder gennant noch sind sie mit beschriebenen Technik herstellbar.

Aufgabe der vorliegenden Erfindung ist es, diastereomere Weinsäuremonoester von optisch aktiven racemischen Alkanolaminen bereitzustellen. Ferner soll erfindungsgemäß ein Weg zur Trennung der optisch aktiven racemischen Alkanolamine gefunden werden, der leicht durchführbar ist und Produkten mit guter optischer Reinheit führt.

Erfindungsgemäß werden Weinsäuremonoester von optisch aktiven, substituierten Alkanolaminen der allgemeinen Formel

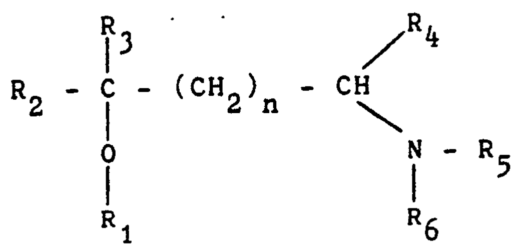

I

sowie deren Salze vorgeschlagen, in der

n null bis vier bedeutet,

$R_1$ (R,R)- oder (S,S)-tartryl bedeutet, in dem der Wasserstoff der Hydroxylgruppen durch eine Acyl-, Alkyl-, Aralkyl-, N-Arylcarbamoyl- oder Alkenylrest, die jeweils substituiert sein können, substituiert ist,

$R_2$ Alkyl, Aralkyl, Aryl, Methylenfluorenonoximether, die Gruppe $R_7$-oxyalkyl, in der $R_7$ Aryl oder eine heterocyclischer Rest ist, wobei die Reste jeweils substituiert sein können, oder Wasserstoff bedeutet,

$R_3$ Aryl, Alkyl, Cycloalkyl, wobei Reste jeweils substituiert sein können, oder Wasserstoff bedeutet,

$R_4$ Alkyl, Aryl, wobei die Reste substituiert sein können, oder Wasserstoff bedeutet,

$R_5$ und $R_6$, die gleich oder verschieden sein können, geradkettiges, verzweigtes oder cyclisches Alkyl, einen 5- oder 6-gliedrigen stickstoffhaltigen Heterocyclus, Aralkyl oder Aminoalkylcarbonyl, wobei die Reste jeweils substituiert sein können, oder Wasserstoff bedeutet, und in der die Reste $R_2$ und $R_5$ oder $R_5$ und $R_6$ ringförmig miteinander verknüpft sein können, wobei die Weinsäuremonoester des Celiprolols ausgenommen sind.

Celiprolol ist ein Alkanolamin der Formel II, in der $R_3$, $R_4$ und $R_5$ Wasserstoff sind, n null, $R_6$ tert. Butyl und $R_2$ die Gruppe

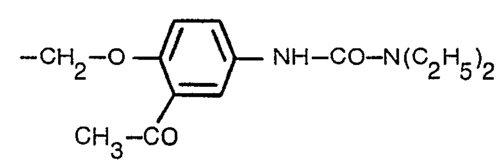

ist.

Die optischen Isomeren der erfindungsgemäßen diastereomeren Weinsäuremonoester weisen überraschend stark unterschiedliche chemische und physikalische Eigenschaften auf, die es nun

ermöglichen, die Isomeren auf einfache Weise in die optisch reinen Verbindungen aufzutrennen. Schließlich kann man die getrennten und nun optisch reinen Weinsäuremonoester auf einfache Weise unter Bewahrung des optisch aktiven Zentrums zu den entsprechenden optischen reinen Alkanolaminen hydrolisieren oder alkoholysieren. Hohe optische Ausbeuten sind erhaltbar, wobei mit dieser Methode gleichzeitig auch die optische Reinheit der Produkte in einfacher Weise kontrolliert werden kann.

Die erfindungsgemäßen Weinsäuremonoester sind somit wertvolle Zwischenprodukte zur Herstellung von Alkanolaminen mit hoher optischer Reinheit.

Ein weiterer Gegenstand der vorliegenden Erfindung sind die optisch reinen Weinsäuremonoester der Formel I, die aus den diastereomeren Weinsäuremonoestern durch Trennung erhalten werden, sowie deren Salze, wobei die Weinsäuremonoester des Celiprolols ausgenommen sind.

In der allgemeinen Formel I ist n vorzugsweise null, eins oder zwei. Besonders bevorzugt ist n null.

$R_2$ ist vorzugsweise ein Alkylrest mit 1 bis 3 C-Atomen, ein Aralkylrest mit 7 bis 10 C-Atomen, Aryl, Methylenfluorenonoximether, die Gruppe $R_7$-oxymethyl, wobei die Reste jeweils substituiert sein können, oder Wasserstoff. Der Alkylrest ist vorzugsweise mit Chlor oder dem Tosyloxyrest substituiert. Der Arylrest ist vorzugsweise die Phenyl-, Indenyl- oder Naphthylgruppe, die jeweils substituiert sein kann. Wenn $R_7$ ein Arylrest ist, ist er vorzugsweise die Phenyl-, Naphthyl-, Indenyl- oder eine Benzocycloalkylgruppe, wie Tetralonyl, die jeweils substituiert sein kann. Besonders bevorzugt ist $R_7$ eine substituierte Phenyl-, Naphthyl- oder Indenylgruppe. Wenn $R_7$ ein heterocyclischer Rest ist, ist er vorzugsweise ein fünf- oder sechsgliedriger Heterocyclus oder ein Benzoheterocyclus, Bevorzugte hereocyclische Reste sind die Thiodiazolyl-, Carbazolyl-, Benzofuranyl-, Indolyl-, Dihydrocarbostyrinlgruppe, die jeweils substituiert sein kann

$R_3$ ist vorzugsweise der Phenyl- oder Cyclohexylrest oder Wasserstoff,

Der $R_4$-Rest ist vorzugsweise Wasserstoff oder ein Alkylrest mit 1 bis 3 C-Atomen.

Die Reste $R_5$ und $R_6$, die gleich oder verschieden sein können, sind vorzugsweise Wasserstoff, ein verzweigter oder geradkettiger Alkylrest mit 1 bis 4 C-Atomen, ein cyclischer Alkylrest mit 4 bis 6 C-Atomen, ein Phenylalkylrest mit 7 bis 12 C-Atomen oder ein Aminoalkylcarbonylrest. Bevorzugt ist $R_5$ Wasserstoff und $R_6$ Wasserstoff, die Ethyl, Propyl-, Isopropyl-, tert. Butyl- oder Aminomethylcarbonylgruppe. $R_6$ kann auch die 1-Phenyl-3-methyl-propyl, Methyl- oder Dimethoxyphenylethylgruppe sein. Besonders bevorzugt sind $R_4$ und $R_5$ Wasserstoff und $R_6$ die Isopropyl- und tert. Butylgruppe.

$R_2$ und $R_5$ können über eine Brücke mit 2 bis 5 C-Atomen miteinander verknüpft sein. Ebenso können $R_5$ und $R_6$ miteinander verknüpft sein.

Besonders bevorzugte Alkanolamine zur Herstellung der erfindungsgemäßen Weinsäuremonoester sind in der Tabelle I angegeben.

Die erfindungsgemäßen Weinsäuremonoester können in Form des diastereomeren Estergemisches hergestellt werden, indem ein racemisches Alkanolamin der allgemeinen Formel

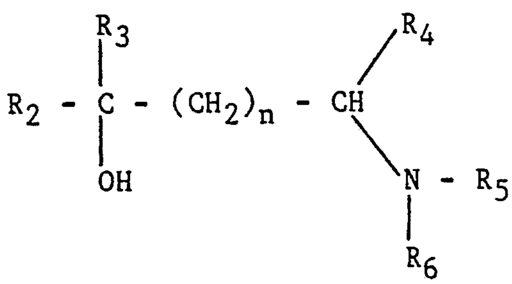

(II)

in der $R_2$ bis $R_6$ un n die oben angegebenen Bedeutungen haben, wobei das Celiprolol ausgenommen ist, mit einer (R,R)- oder (S,S)-O,O-disubstituierten Weinsäure oder mit deren Anhydrid in der Schmelze oder in einem aprotischen Lösungsmittel umgesetzt wird, wobei im Falle der Verwendung der freien disubstituierten Weinsäure in Gegenwart eines Kondensationsmittels gearbeitet wird.

Zur Herstellung der erfindungsgemäßen Weinsäuremonoester werden O,O-disubstituierte (R,R) oder (S,S)-Weinsäure oder deren Anhydride verwendet. Die Substituenten für den Wasserstoff der Hydroxylgruppen sind der Acyl-, Alkyl-, Aralkyl-, N-Arylcarbamoyl- oder Alkenylrest, die jeweils substituiert sein können. Als Acylreste in den O,O-Diacylweinsäuren kommen insbesondere ein ggf. substituierter Alkanoylrest und ein ggf. substituierter Aroylrest sowie ein Cycloalkanoylrest infrage. Der Alkanoylrest kann beispielsweise mit einem Arylrest oder Halogen substituiert sein, wobei als Beispiel der Trichloroacetylrest, der Phenylacetylrest und der Dinitrophenylacetylrest, hervorzuheben sind. Bevorzugt sind unsubstituierte Alkanoylreste mit 1 bis 4 C-Atomen, wobei wiederum der Acetylrest besonders bevorzugt ist. Als Aroylrest kommen neben dem Benzoylrest der Toluoyl-, Nitrobenzoyl- und Dinitrobenzoylrest infrage, wobei der Benzoyl- und Toluoylrest besonders bevorzugt sind. Als Cycloalkanoylrest kommt insbesondere der Cyclohexylcarbonylrest in Betracht. Als Alkylreste in den O,O-Dialkylweinsäuren werden vorzugsweise Alkylreste mit 1 bis 5 C-Atomen verwendet, besonders bevorzugt mit 1 bis 4 C-Atomen, wobei die Methyl-, Ethyl- und tert. Butylgruppe wiederum besonders bevorzugt sind, die jeweils substituiert sein können. Als Aralkylrest ist der Benzylrest bevorzugt. Als Alkenylrest kommen vorzugsweise Gruppen mit 2 bis 5 C-Atomen infrage.

Besonders bevorzugt sind der Vinyl- und Allylrest, die mit Halogen und insbesondere mit Chlor substituiert sein können. Als N-Arylcarbamoylrest sind besonders der N-Phenylcarbamoylrest und der N-Naphthylcarbamolyrest zu nennen.

Als Kondensationsmittel kommen Dicyclohexylcarbodiimid, Tosylchlorid, Trifluoressigsäureanhydid, Sulfurylchlorid, ein Gemisch von Dimethylformamid und Thinoylchlorid, Aluminiumoxid sowie Molekularsiebe infrage, wobei die Carbodiimid-Methode bevorzugt ist.

Vorzugsweise erfolgt die Umsetzung mit dem Anhydrid der Säuren in einem aprotische Lösungmittel bei Temperaturen von Raumtemperatur bis 150°C. Die jeweils verwendete Temperatur hängt von dem verwendeten Lösungsmittel sowie den Reaktionspartnern ab. Sie liegt vorzugsweise im Bereich von Raumtemperatur bis 90°C, insbesondere 40° bis 90°C. Die Säuren bzw. Säurederivate werden äquimolar oder im Überschuß eingesetzt.

Als aprotische Lösungsmittel können bevorzugt Dichlormethan, 1,2-Dichlorethan, Aceton, Acetonitril, Toluol, Dimethylformamid, Dimethylsulfoxid, Tetrahydrofuran, Dioxan, Ethylenglykoldimethylethan oder N-Methyl-pyrrolidon-2 verwendet werden. Die Lösungsmittel werden in wasserfreiem Zustand eingesetzt.

Auch wenn die Aminfunktion von racemischen Alkanolaminen mit primärer oder sekundärer Aminogruppe ($R_5$ und/oder $R_6$ Wasserstoff) nicht geschützt wird, entstehen bei der Umsetzung mit optisch reinen O,O-disubstituierten Weinsäurederivaten, z.B. den Annydriden, überraschenderweise bevorzugt die diastereomeren Monoester, währen die Monoamide un doppelt substituierten Derivate nur als Nebenprodukte entstehen. Aus solchen Reaktionsgemischen können die Monoester relativ leicht isoliert werden.

Vorzugsweise wird man jedoch die Aminfunktion im Falle von primären und sekundären Aminogruppen bei der Umsetzung schützen, wozu jede für Aminogruppen übliche Schutzgruppe geeignet ist, die durch Verseifung oder Hydrierung abgespalten werden kann. Die Abspaltung kann unmittelbar nach der Veresterungsreaktion oder nach der Auftrennung des Isomerengemisches der Weinsäuremonoester erfolgen. Die Aminogruppen können auch gleichzeitig bei der hydrolytischen Spaltung der Weinsäuremonoester zur Gewinnung der optisch reinen Alkanolamine in die freie Form überführt werden. Vorzugsweise wird die Aminogruppe durch Salzbildung geschützt. Zur Salzbildung werden starke anorganische oder organische Säuren verwendet, die mit der Aminogruppe eine Ionenpaarbindung eingehen, die in dem aprotischen Lösgungsmittel nur schwach dissoziiert. Als organische Säuren kommen z.B. Sulfonsäuren und Halogencarbonsäuren, als anorganische Säuren kommen vor allem Mineralsäuren, wie Halogenwasserstoffsäuren, infrage. Vorzugsweise verwendet man Toluolsulfonsäure, Trichloressigsäure und Trifluoressigsäure und Salzsäure. Die durch Salzbildung geschützten Aminogruppen können unmittelbar nach der Veresterungsreaktion durch Wasser oder durch schwache Basen, wie z.B. wäßrige $NaHCO_3$-Lösungen, in die freie Form überführt werden.

Die gebildeten Weinsäuremonoester der Alkanolamine können aus wäßrigem Medium, bevorzugt im pH-Bereich von 0 bis 9, kristalliert oder mittels organischer Lösungsmittel, bevorzugt Dichlormethan oder Dichlorethan, extrahiert werden.

Aus dem erhaltenen Isomerengemisch der diastereomeren Ester können die optisch reinen Isomeren erhalten werden, indem das isomerengemisch einer selektiven Kristallisation oder Extraktion unterworfen oder auf chromatographischem Weg getrennt wird.

Bei der fraktionierten Kristallisation wird ein Lösungsmittel gewählt, das die isomeren unterschiedlich gut löst. Als Lösungsmittel kommen Chlorkohlenwasserstoffe, Ketone, wie Aceton, Ether, Ester, sec. und tert. Alkohole, wie Isopropanol, oder Wasser bzw. wäßrige Pufferlösungen infrage. Das jeweils geeignetste Lösungsmittel hängt von dem betreffenden Weinsäureester ab. Meist gelangt man nach bereits 1 bis 4 Kristallisationsschritten zu optisch reinen Weinsäureestern.

Vorzugsweise erfolgt die Trennung durch Extraktion oder chromatographisch. Für die Extraktion wird ein Extraktionsmittel verwendet, in dem die Ester nicht oder nur langsam solvolysierbar sind. Als Extraktionsmittel kommen Chlorkohlenwasserstoffe, Ketone, Ether, Ester, sec. und tert. Alkohole oder Wasser (wäßrige Pufferlösung) infrage. Vorzugsweise werden Aceton, Isopropanol Dichlormethan, Dichlorethan, Chloroform, Essigester, Cyclohexan-Toluol, Tetrahydrofuran, Dioxan, verschiedene Ether, wie z.B. Methyl-tert. butylether, oder wäßrige organische oder anorganische Pufferlösungen, wie Alkali- oder Ammoniumphosphatpuffer, verwendet.

Als chromatographische Trennverfahren sind sowohl die Dünnschichtchromatographie, die Säulenchromatographie (LC) als auchy die Hochdruckflüssigkeits-Chromatographie (HPLC) geeignet. Bevorzugt sind die Adsorptionschromatographie an Kieselgelsäulen oder Aluminiumoxidsäulen mit organischen Laufmitteln, die nicht oder nur schwach die Weinsäuremonoester solvolysieren, wie z.B. Aceton, Isopropanol, Dichlormethan, Cyclohexan oder Toluol, vorzugsweise Aceton/Isopropanol, und die Hockdruckflüssigkeitschromatographie mit Reversed Phase-System, z.B. mit RP 18 (Reversed Phase-Octadecyl) als stationäre Phase. Als mobile Phase können im letzteren Fall saure Puffer mit Methanol oder Acetonitril gemischt dienen.

In der Flüssig-Chromatographie (LC oder HPLC) erreicht man Stofftrennungen aufgrund unterschiedlicher chemischphysikalischer Wechselwirkungen innerhalb eines 3-Compartment Systems, den zu trennenden Substanzen, der stationären Phase und der mobilen Phase. In einem Reversed-Phase-System beruht die Auftrennung eines Gemisches z.B. bevorzugt aufgrund unterschiedlicher Lipophilie der Substanzen in der mobilen und stationären Phase, was auch durch unterschiedliche Verteilungskoeffizienten zu

beschreiben ist. Unter konstanten isokratischen und isothermen Bedingungen (definierte stationäre Phase, z.B. RP 18, und definierte mobile Phase, z.B. bestimmtes Methanol-Puffer Gemisch) wird das Retentionszeitverhalten einer Substanz über inhre Gesamt-retentionszeit beschrieben. Diese ist von diversen Säulenparametern und der Fließgeschwindigkeit der mobilen Phase abhängig. Um eine weitgehend unabhängige Beschreibung des Substanzverhaltens in einem Chromatographiesystem zu erhalten, formulierte man den Kapazitätsfaktor $k_i'$.

$$k_i' = \frac{t_{ri} - t_o}{t_o}$$

i = Substanz i; $t_{ri}$ = Gesamtretentionszeit der Substanz i; $t_o$ = Totzeit einer nicht retardierten Substanz im gleichen chromatogr. System

Um das Verhalten zweier Substanzen in ein und demselben Chromatographie-System auf einfache Weise zu beschreiben, definierte man die "Relative Retention", oder auch "Selektivitätsfaktor α", der somit die Auftrennbarkeit eines Substanzgemisches i-j angibt

$$\alpha_{i,j} = \frac{k_j'}{k_i'}$$ (Substanz j wird länger retardiert als Substanz i.)

Hohe α-Werte bedeuten somit eine sehr selektive Auftrennung eines bestimmten Substanzgemisches, welches in den nachfolgenden Beispielen jeweils ein diastereomeres Isomerenpaar darstellt. Die k' und α-Werte stellen somit chemisch-physiskalische Größen dar, welche substanzspezifisch und substanzcharackteristisch in einem gegebenen Chromatographiesystem (stationäre Phase in Kombination mit einer bestimmten mobilen Phase) sind. (Literatur: Chromatogr. Trennmethoden, G. Schwendt, Georg Thieme Verlag Stuttgart, 1979).

Die auf einem der genannten Wege getrennten optisch reinen Alkanolaminweinsäuremonoester können unter sauren Bedingungen, z.B. mit 0,1 bis 1 n wäßriger oder ethanolischer Salzsäure, oder unter basischen Bedingungen, z.B. mit 0,1 bis 1 n wäßriger oder methanolischer NaOH, oder durch Umesterung z.B. in Methanol im Temperaturbereich zwischen −10° und 40°C solvolytisch gespalten werden.

Eine andere Möglichkeit zur Herstellung der optisch reinen Alkanolamine besteht darin, daß man das Gemisch der diastereomeren Alkanolaminweinsäuremonoester einer stereospezifischen, pH-kontrollierten Hydrolyse oder einer stereospezifischen enzymatischen Hydrolyse unterwirft. Der oben aufgezeigte Weg über die Trennung der Weinsäuremonoester mit anschließender Hydrolyse ist jedoch bevorzugt.

Die Erfindung wird anhand der folgenden Beispiele näher erläutert.

Beispiel 1

Herstellung von (R,S)-Propranolol-(R,R)-O,O-Diacetylweinsäuremonoester:

25,9 g (0,1 Mol) trockenes (R,S)-Propanolol bas. werden mit 20,0 g (0,105 Mol) p-Toluolsulfonsäure·$H_2O$ in 300 ml 1,2-Dichlorethan bei Raumtemperatur gelöst und das Kristallwasser der Toluolsulfonsäure durch Abdestillieren entfernt. Zu der filtrierten Lösung werden 32,4 (0.15 Mol) (R,R)-(+)-O,O-Diacetylweinsäureanhydrid bei Raumtemperatur zugegeben und die Mischung dann am Rückfluß bei ca. 80°C 24 h gerührt. Nach Entfernung des Lösungsmittels im Vakuum wird der feste Rückstand in 5% waßriger $NaHCO_3$-Lösung bis zur Lösung aufgenommen (ca. 200 ml) und anschließend mit In HCl bis zu einem pH von 2,5 acidifiziert. Das Diastereomerengemisch des (R,S)-Propanolol-(R,R)-O,O-Diacetylweinsäuremonoesters fällt aus bzw. wird mit $CH_2Cl_2$ (3 × 100 ml) extrahiert. Nach Entfernung des Extraktionsmittels erhält man einen Rückstand (Ausbeute 38 g, 80% der Theorie), welcher in Aceton zur Kristallisation gerbracht wird.

Obiger Ansatz ist unter völlig gleichen Bedingungen auch mit (S,S)-(−)-O,O-Diacetylweinsäureanhydrid als Reagens durchführbar, und man erhält ebenfalls 80% Ausbeute des entsprechenden Diastereomerengemisches. Zur Auftrennung der Gemische siehe Beispiele 3 bis 8.

Beispiel 2

Herstellung von (R,S)-Propranolol-(R,R)-O,O-Diacetylweinsäuremonoester:

1,81 g (7 mM) trockenes (R,S)-Propranolol bas. werden in 20 ml Dichlormethan gelöst und bei Reaumtemperatur mit 3,02 g (14 mM) (R,R)-(+)-O,O-Diacetylweinsäureanhydrid versetz und 48 Stunden bei Raumtemperatur gerührt. Nach Entfernung des Lösungsmittels im Vakuum wird der Rückstand mit 16 mM $NaHCO_3$, gelöst in 50 ml Wasser, unter Rühren und bei Raumptemperatur versetzt, wobei ein kristalliner Niederschlag anfällt. Dieser wird abgesaugt, mit $H_2O$ gewaschen und stellt reinen (S)-Propranolol-(R,R)-O,O-Diacetylweinsäuremonoester (vgl. Fraktion II Beispiel 3) dar, welcher bei der Hydrolyse optisch reines (>98%) (S)-(−)-Propranolol ergibt. Die Lösung enthält ein Gemisch von diastereomerem (R)- und (S)-Propranolol-(R,R)-O,O-Diacetylweinsäuremonoester und kann nach einem der Beispiele 3 bis 8 in die optischen Isomeren aufgetrennt werden.

Ausbeute an Fraktion II: 0,7 g, 42% der Theorie.

Beispiel 3

Trennung das nach Beispiel 1 erhaltenen Gemisches der diastereomeren (R)- und (S)-Propranolol-(R,R)-O,O-Diacetylweinsäuremonoester mittels Extraktion in die optischen Isomeren:

15 g der aus dem Diastereomerengemisch bestehenden Kristallmasse werden in einer Soxhlet-Apparatur in der Hülse vorgelegt und mit Aceton wiederholt und unter Rühren extrahiert. Nach ca. 10 Überführungen bei einem Lösungsmittelvolumen von ca. 300 ml, kristallisiert in der Vorlage reiner (S)-Propranolol-(R,R)-O,O-Diacetylweinsäuremonoester (Fraktion II) aus, wohingegen reiner (R)-Propranolol-(R,R)-O,O-Diacetylweinsäuremonoester (Fraktion I) in der Soxhlethülse zurückbleibt.

Fraktion I:

(R)-Propranolol-(R,R)-O,O-Diacetylweinsäuremonoester

Ausbeute 7g; Schmelzpunkt 196°—198°C.

$[\alpha]^{22}_{546} = +13{,}5°$ (c = 0,405 in DMSO) optische Reinheit >98%

Fraktion II:

(S)-Propranolol-(R,R)-O,O-Diacetylweinsäuremonoester

Ausbeute 5 g; Schmelzpunkt 176°—177°C.

$[\alpha]^{22}_{546} = +33{,}3°$ (c = 0,98 in DMSO) optische Reinheit >98%.

In der Aceton-Mutterlauge verbleiben 3 g eines Gemisches aus Fraktion I und Fraktion II.

Spektrokopische Daten:

$^{13}C$ NMR, XL 200·DMSO $d_6$,

TMS als Innerer Standard,

δ (ppm)

| | Fraktion I | Fraktion II | (R,S)-Propanolol. HCl (in $D_2O$) |
|---|---|---|---|
| =*CH—*CH= | 73,39 ; 72,68 | 74,92 ; 73,11 | — |
| =*CH—O— | 69,11 | 69,11 | 62,37 |
| —$CH_2$—O— | 67,0 | 67,13 | 66,45 |
| =CH—NH— | 48,84 | 49,72 | 47,92 |
| —$CH_2$—NH— | 43,18 | 44,46 | 43,46 |

Formelschema A(R)- bzw. (S)Propranolol-(R,R)-O,O-Diacetylweinsäuremonoester (Fraktion I bzw. Fraktion II)

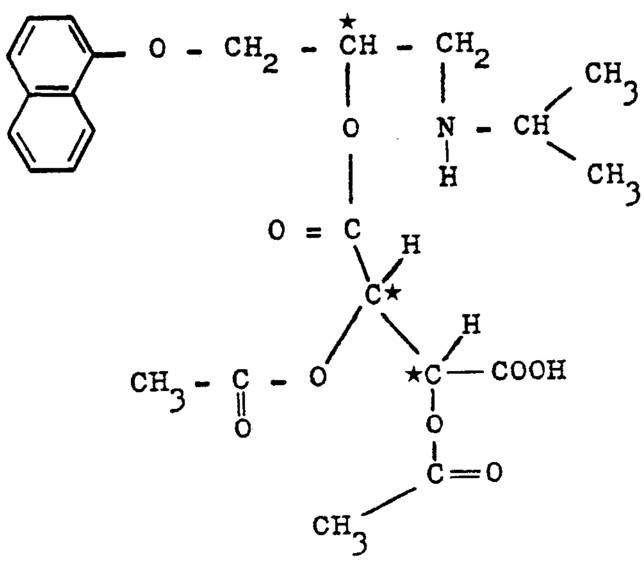

MG 475,48

Die bei der Umsetzung von (R,S)-Propranolol mit (S,S)--O,O-Diacetylweinsäure-anhydrid, statt (R,R)-O,O-Diacetylweinsäure-anhydrid, erhältlichen Weinsäuremonoester verhalten sich in ihrer Lipophilie bzw. Extrahierbarkeit invers zu vorstehendem Aufarbeitungsschema, d.h. der Soxhletrückstand ist dann (S)-Propranolol-(S,S)-O,O-Diacetylweinsäuremonoester, wohingegen die leichter extrahierbare Fraktion der (R)-Propranolol-(S,S)-O,O-Diacetylweinsäuremonoester ist. Derartige Inversionseigenschaften diastereomerer Verbindungen haben Allgemeingültigkeit und gelten somit auch für alle anderen (R,R)- bzw. (S,S)-O,O-

Diacetylweinsäurederivate von Alkanolaminen. Auch für die Analytik hat dies Bedeutung, denn unter Zuhilfenahme dieser Regel gelingt es relativ einfach, zusammenhängende Isomerenpaare aufgrund der Inversion deren Peakverhältnisse zu identifizieren.

Aufgrund der chemischen Struktur der Alkanolaminweinsäuremonoester liegen dises zwitterionisch vor bzw. bilden innere Salze.

Mit starken Säuren, z.B. Salzsäure, können auch die Hydrochloride in nichtwäßrigen aprotischen Lösungsmitteln hergestellt werden.

Ebenso können Alkali- oder Erdalkali-Salze der Carboxylfunktion durch äquivalente Zugabe von Alkali- oder Erdalkalihydroxiden hergestellt werden.

Beispiel 4

Trennung des nach Beispiel 1 erhaltenen Gemisches der diastereomeren (R)- und (S)-Propranolol-(R,R)-O,O-Diacetylweinsäuremonoester in die optischen Iosmeren mittels Dünnschicht-Chromatographie:

DC-Platte:

Kieselgel Si 60 (Merck); Laufmittel: Aceton Isopropanol 1:1

Entwicklungsstrecke: 8cm

$$R_f \text{ Werte: } \frac{\text{Fraktion II } 0{,}41}{\text{Fraktion I } 0{,}23} = 1{,}78$$

Die Dünnschicht-Chromatographie auf Kieselgel kann uneingeschränkt als Pilottechnik für die präparative und analytische Säulenchromatographie, mit Kieselgel als Adsorbens, angesehen werden, wobei zumindest ähnliche Selektivitätsfaktoren zu erhalten sind.

Beispiel 5

Trennung der Gemische der diastereomeren (R,R)-O,O-Diacetylweinsäuremonoester der in der Tabelle II angegebenen (R,S)-Alkanolamine (Formelschema siehe Tabelle I) mittels Umkehr-Phase-Chromatographie (Reversed Phase HPLC):

Die Umsetzung der (R,S)-Alkanolamine mit (R,R)-O,O-Diacetylweinsäureanhydrid wird gemäß den Beispielen 1 oder 2, jedoch für analytische Zwecke in geringeren Mengen und wahlweise mit den wasssserfreien Lösungsmitteln Dichlormethan, Dichlorethan, Tetrahydrofuran, Aceton, Acetonitril, Dioxan, durchgeführt, wobei die jeweiligen Lösungsmittel nach der Reaktion entfernt werden, und der Rückstand in Methanol aufgenommen wird. Sofern Schutzgruppen angewendet werden, dienen wahlweise die Säuren Toluolsulfonsäure, Trichlor- bzw. Trifluoressigsäure.

Die methanolischen Lösungen der jeweils erhaltenen Gemische der diastereomeren Weinsäuremonoester werden direkt auf die Chromatographiesäule gegeben. Die Reaktionstemperatur liegt in Abhängigkeit der Siedepunkte der Lösungsmittel zwischen Raumtemperatur und 80°C und die Reaktionszeiten zwischen 0,2 und 36 Stunden.

Chromatographisches Trennsystem:

Säule 250 × 4,6 mm I.D. gepackt mit Spherisorb RP 18, 5 µm.

Mobile Phase:

2% Essigsäure in $H_2O$ bidest. mit $NH_3$ auf pH 3,7 eingestellt — Methanol/50-50; Flußrate: 1,5 ml/min.

| Ester von (R) bzw. (S) | $k'_R$ | $k'_S$ | $\alpha = \frac{k'_S}{k'_R}$ |
|---|---|---|---|
| Propranolol | 2,5 | 6 | 2,4 |
| Oxprenolol | 0,7 | 1,6 | 2,3 |
| Metoprolol | 0,67 | 1,67 | 2,5 |
| Alprenolol | 0,7 | 1,6 | 2,3 |
| Carazolol | 0,7 | 1,5 | 2,14 |

Beispiel 6

Trennung der Gemische der diastereomeren (R,R)-O,O-Dibenzoylweinsäuremonoester der in der Tabelle II angegebenen (R,S)-Alkanolamine mittels Reversed-Phase-HPLC:

Die Umsetzung der (R,S)-Alkanolamine mit (R,R)-O,O-Dibenzoylweinsäureanhydrid wird gemäß dem Beispiel 5 durchgeführt.

Chromatographisches Trennsystem:

Säule wie Beispiel 5; Mobile Phase: 2% Essigsäure in $H_2O$ bidest. mit $NH_3$ auf pH 3,7 eingestellt — Methanol/35—65.

TABELLE III

| Ester von (R) bzw. (S) | $k'_R$ | $k'_S$ | $\alpha = \frac{k'_S}{k'_R}$ |
|---|---|---|---|
| Oxprenolol | 2,38 | 6,81 | 2,87 |
| Propranolol | 4,0 | 11,0 | 2,75 |
| Metoprolol | 1,38 | 4,63 | 3,35. |
| Acebutolol | 0,88 | 2,75 | 3,13 |
| Bunitrolol | 0,88 | 3.13 | 3,55 |
| Methypranolol | 2,13 | 8,38 | 3,93 |
| Alprenolol | 2,38 | 6,38 | 2,68 |
| Pindolol | 1,88 | 4,88 | 2,59 |
| Carazolol | 2,38 | 6,38 | 2,68 |
| Midodrin | 0,63 | 4,38 | 6,9 |
| Nifenalol | 0,63 | 3,0 | 4,8 |
| Norpropranolol | 1,63 | 4,0 | 2,5 |
| Bupranolol | 3,25 | 10,88 | 3,35 |
| Atenolol | 0,35 | 1,1 | 3,11 |
| Labetalol | 1,28 | 6,39 | 4,99 |
| Timolol | 2,33 | 6,22 | 2,67 |

Beispiel 7

Trennung des Gemisches des diastereomeren (R,R)-O,O-Ditoluoylweinsäuremonoesters von (R,S)-Propranolol mittels Reversed-Phase-HPLC:

Die Umsetzung des (R,S)-Propranolols mit (R,R)-O,O-Ditoluoylweinsäureanhydrid wird gemäß dem Beispiel 5 durchgeführt.

Chromatographisches Trennsystem:

Säule wie Beispiel 5; Mobile Phase: wie Beispiel 6.

| Ester von (R) bzw. (S) | $k'_R$ | $k'_S$ | $\alpha = \frac{k'_S}{k'_R}$ |
|---|---|---|---|
| Propranolol | 7,1 | 21,1 | 2,98 |

Beispiel 8

Trennung der Gemische der diastereomeren (R,R)-O,O-Dimethylweinsäuremonoester der in der Tabelle IV angegebenen (R,S)-Alkanolamine mittels Reversed-Phase-HPLC:

Die Umsetzung der (R,S)-Alkanolamine mit (R,R)-O,O-Dimethylweinsäureanhydrid wird gemäß dem Beispiel 5 durchgeführt.

Chromatographisches Trennsystem:

Säule wie in Beispiel 5; Mobile Phase: 2% AmmonAc (ph 3,7) — MeOH/65—35.

TABELLE IV

| Ester von (R) bzw. (S) | $k'_R$ | $k'_S$ | $\alpha = \frac{k'_S}{k'_R}$ |
|---|---|---|---|
| Propranolol | 5,8 | 8,7 | 1,5 |
| Oxprenolol | 1,5 | 2,17 | 1,4 |
| Pindolol | 0,94 | 1,33 | 1,41 |
| Metoprolol | 2,8 | 4,0 | 1,42 |
| Norpropanol | 6,11 | 10,22 | 1,67 |
| Labetalol | 6,05 | 8,33 | 1,37 |
| Bunitrolol | 1,61 | 2,16 | 1,34 |
| Alprenolol | 5,05 | 7,0 | 1,38 |
| Nifenalol | 0,38 | 0,72 | 1,89 |
| Acebutolol | 1,61 | 2,61 | 1,62 |
| Carzolol | 4,22 | 6,77 | 1,6 |
| Timolol | 2,33 | 3,16 | 1,35 |
| Oxprenolol | 5,11 | 7,0 | 1,37 |
| Methypranolol | 9,0 | 14,1 | 1,57 |
| Mobile Phase 50—50 | | | |
| Propranolol | 2,18 | 3,37 | 1,54 |
| Pindolol | 0,66 | 1,11 | 1,68 |
| Mobile Phase 80—20 | | | |
| | $k'_1$ | $k'_2$ | $\alpha = \frac{k'_2}{k'_1}$ |
| 1-Amino-propanol-2 | 0,66 | 1,55 | 2,35 |
| Mobile Phase 90—10 | | | |
| | $k'_R$ | $k'_S$ | $\alpha = \frac{k'_S}{k'_R}$ |
| Atenolol | 6,55 | 9,33 | 1,42 |
| Sotalol | 2,66 | 4,11 | 1,54 |

Beispiel 9

Hydrolytische Spaltung von (R)-Propranolol-(R,R)-O,O-Diacetylweinsäuremonoester:

5 g (R)-Propranolol-(R,R)-O,O-Diacetylweinsäuremonoester (vgl. Fraktion I von Beispiel 3) werden bei Raumtemperature in 50 ml methanolische NaOH (1,5 g NaOH Plätzchen gelöst in 50 ml Methanol) eingetragen. Nach einer Stunde Reaktionsdauer wird der entstandene Niederschlag abgetrennt und die Mutterlauge im Vakuum zur Trockene eingeengt. Der Rückstand wird mit ln wäßriger HCl gelöst und mit Ammoniak bis zu einem pH von 9,0 versetzt. Reines (R)-(+)-Propranolol fällt aus.

Ausbeute 2,5 g (88% de Theorie); Schmelzpunkt 73°C
$[\alpha]_D^{20}$ + 8,33° (c = 1,0 in EtOH 95%)
Optische Reinheit >98%.

Beispiel 10

Hydrolytische Spaltung von (S)-Propranolol-(R,R)-O,O-Diacetylweinsäuremonoester:

2,5 g (S)-Propranolol-(R,R)-O,O-Diacetylweinsäuremonoester (vgl. Fraktion II von Beispiel 3) werden in 30 ml ca. In methanolischer Salzsäure bein Raumtemperatur gelöst und 24 h stehen gelassen. Die Lösung wird im Vakuum zur Trockene eingeengt, der Rückstand in Wasser aufgenommen und mit Ammoniak versetzt, wobei reines (S)-(−)-Propranolol ausfällt.

Ausbeute 1,2 g (85% de Theorie); Schmelzpunkt 72° bis 73°C
$[\alpha]_D^{20}$ + 8,32° (c = 1,0 in EtOH 95%)
Optische Reinheit >98%.

Beispiel 11

Herstellung von (R,S)-Propranolol-(R,R)-O,O-Diacetylweinsäuremonoester:

2,34 g (10 mMol) (R,R)-O,O-Diacetylweinsäure werden in 15 ml Dichlormethan suspendiert und mit 1,72 g (10 mMol) wasserfreier Toluolsulfonsäure versetzt. Dazu wird unter Rühren eine Lösung von 1,3 g (R,S)-Propranolol (5 mMol) in 10 ml Dichlormethan getropft und anschliessend eine Lösung von 2,06 g Dicyclohexylcarbodiimid in 15 ml Dichlormethan. Es wird 24 Stunden bei Raumtemperatur gerührt, dann wird abgesaugt und das Filtrat mit 15 ml und 10 ml Wasser gewaschen. Die organische Phase wird getrocknet und das Lösungsmittel am Rotationsverdampfer abgezogen; der Rückstand wird mit wäßriger $NaHCO_3$-Lösung behandelt. Die filtrierte wäsrige Lösung wird auf pH 3 angesäuert, und das Diastereomerengemisch des Weinsäureesters wird mit Dichlormethan extrahiert, welches man danach einengt und den Rückstand in Aceton suspendiert. Die weitere Aufarbeitung des Reaktionsproduktes zur Trennung des Diastereomerengemisches in die Fraktionen I und II kann gemäß der Beispiele 3 bis 8 erfolgen.

Beispiel 12

Herstellung von (R,S)-Metoprolol-(R,R)-O,O-Dibenzoylweinsäuremonoester:

7 g (26 mMol) (R,S)-Metoprolol bas. werden gemeinsam mit 4,4 g (27 mMol) Trichloressigsäure in 150 ml 1,2-Dichlorethan bei Raumtemperatur gelöst. Am Rotationsverdampfer wird azeotrop eine Fraktion von ca. 30 ml abgedampft und dient der Entwässerung. Zur verbleibenden Lösung gibt man 18 g (53 mMol) (R,R)-O,O-Dibenzoylweinsäureanhydrid und erwärmt die Lösung auf ca. 80°C für 2 Stunden. Nach Abkühlung filtriert man die Lösung von überschüssigem Reagens ab und engt die verbleibende Lösung am Rotationsverdampfer zur Trockene ein. Der Rückstand wird mit 50 ml Aceton und 100 ml wäßrige 10% $NaHCO_3$-Lösung aufgenommen, wobei sich ein Niederschlag bildet, welcher ein Gemisch aus den Weinsäuremonoestern von (R)- und (S)-Metoprolol darstellt.

Nach dem Abnutschen und Waschen des Niederschlags mit $H_2O$ wird dieser in 100 ml Aceton suspendiert und ca 1 Stunde bei Raumtemperatur gerührt. Der Niederschlag wird wiederum abfiltriert und stellt oben genanntes Estergemisch dar, wobei der Anteil an (R)-Metoprolol-(R,R)-O,O-Dibenzoylweinsäuremonoester (Fraktion A) überwiegt. Der korrespondierende (S)-Metoprololester (Fraktion B) liegt angereichert in der Acetonphase vor, welche zur Trockene eingeengt wird.

Die Reinigung der enzelnen Metoprolol Fraktionen A) und B) erfolgt nach Beispiel 13.

Gesamtausbeute an Fraktion A) gemeinsam mit Fraktion B): 12,8 g (80% der Theorie).

Formelschema: (R)- bzw. (S)-Metoprolol-(R,R)-O,O-Dibenzoylweinsäuremonoester (Fraktion A) bzw. Fraktion B))

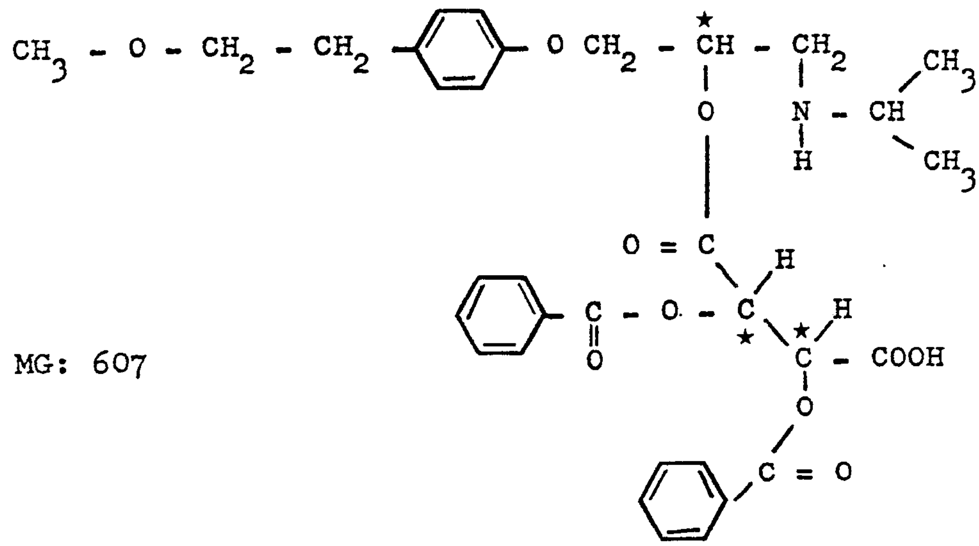

MG: 607

Beispiel 13

Trennung des Diastereomerengemisches von (R)- und (S)-Metoprolol-(R,R)-O,O-Dibenzoylweinsäuremonoester in die optischen Isomeren mittels Adsorptions-Chromatographie:

5 g Gemisch aus Fraktion A) und Fraktion B) (siehe Beispiel 12) werden in 20 ml Aceton gelöst und auf eine präp. Kieselgelsäule (Inhalt ca. 350 g Kieselgel 70—230 mesh), welche in Aceton vorliegt, aufgebracht und mit Aceton eluiert. Fraktion B) eluiert vor Fraktion A), und es gelingt auf diese Weise, in guter Ausbeute Fraktion B) mittels Fraktionsschneidens rein zu isolieren.

Fraktion B): (S)-Metoprolol-(R,R)-O,O-Dibenzoylweinsäuremonoester.

Ausbeute: 1,1 g

$[\alpha]_{546}^{31} = -57{,}2°$ (c = 1 $CHCl_3$) optische Reinheit >98%

$^1H$—NMR (DMSO-$d_6$), δ, ppm, TMS als Innerer Standard.

| | asymmetrische-CH der Weinsäure | Isopropyl-CH |
|---|---|---|
| Fraktion A | 6.30 (d, 1H, 3Hz); 5.91 (d, 1H, 3Hz) | 3.90 (m, 2H, $CH_2O$) |
| Fraktion B | 5.45 (d, 1H, 8Hz); 5.33 (d, 1H, 8Hz) | 4.16 (m, 2H; $CH_2O$) |

Beispiel 14

Trennung des Diastereomerengemisches von (R)- und (S)-Metoprolol-(R,R)-O,O-Dibenzolylweinsäuremonoester in die optischen Isomeren mittels Dünnschicht-Chromatographie:

DC-Platte: Kieselgel
Laufmittel: $R_f$ Werte

| | Fraktion A | Fraktion B | |
|---|---|---|---|
| Aceton | 0,09 | 0,21 | 2,3 |
| Aceton-Dioxan/9:1 | 0,16 | 0,34 | 2,1 |
| Aceton-Dioxan/1:1 | 0,54 | 1 | 1,9 |

Beispiel 15

Hydrolytische Spaltung von (S)-Metoprolol-(R,R)-O,O-Dibenzoylweinsäuremonoester;

2,3 g (S)-Metoprolol-(R,R)-O,O-Dibenzoylweinsäuremonoester ester (vgl. Beispiel 13, Fraktion B) werden bei Raumtemperatur in 70 ml methanolischer KOH (0,84 g KOH Plätzchen, gelöst in 70 ml Methanol) eingetragen und 30 Minuten gerührt. Danach wird die Lösung mit Salzsäure auf einen pH-Wert von 7,0 gebracht und das Methanol am Rotationsverdampfer abgezogen. Der Rückstand wird mit 70 ml Wasser aufgenommen, mit verdünntem Ammoniak basifiziert und die Lösung mit Ether extrahiert. Aus der getrockneten Etherlösung erhält man reines (S)-(−)-Metoprolol (bas.).

$[\alpha]_{546}^{31} = -9{,}0$ (c = 1,0 $CHCl_3$)

Beispiel 16

Trennung der Gemische der diastereomeren (R,R)-O,O-Dibenzylweinsäuremonoester der in der Tabelle V angegebenen (R,S)-Alkanolamine mittels Reversed-Phase-HPLC:

Die Umsetzung der Alkanolamine mit (R,R)-O,O-Dibenzylweinsäureanhydrid wird gemäß Beispiel 5 durchgeführt.

Chromatographisches Trennsystem:

Mobile Phase: 2% Essigsäure in $H_2O$ bidest. mit $NH_3$ aus pH 3,7 eingestellt — Methanol/65—35 Säule wie in Beispiel 5.

TABELLE V

| Ester von (R) bzw. (S) | k'$_1$ | k'$_2$ | $\alpha = \frac{k'_2}{k'_1}$ |
|---|---|---|---|
| 2-Amino-1-propanol | 10,8 | 21,0 | 2,13 |
| 1-Amino-2-propanol | 2,88 | 4,77 | 1,66 |
| 3-Hydroxy-pyrrolidin | 4,55 | 6,77 | 1,49 |
| 3-Hydroxy-N-methylpiperidin | 4,77 | 7,33 | 1,53 |

Mobile Phase 50—50

| | k'$_R$ | k'$_S$ | $\alpha = \frac{k'_S}{k'_R}$ |
|---|---|---|---|
| Acebutolol | 9,55 | 20,11 | 2,1 |

Beispiel 17

Reversed-Phase HPLC Trennung der diasteromeren (R,R)-O,O-Diethylweinsäuremonoester der in der Tabelle VI angegebenen (R,S)-Alkanolamine:

Die Umsetzung der (R,S)-Alkanolamine mit der (R,R)-O,O-Diethylweinsäureanhydrid wird gemäß dem Beispiel 5 durchgeführt.

Chromatographisches Trennsystem:

Säulen wie in Beispiel 5;

Mobile Phase: 0,1 M Phosphorsäure mit wäßriger $NH_3$ auf pH 3,6 eingestellt — MeOH/50—50.

TABELLE VI

| Ester von (R) bzw. (S) | k'$_R$ | k'$_S$ | $\alpha = \frac{k'_S}{k'_R}$ |
|---|---|---|---|
| Propranolol | 5,45 | 11,45 | 2,1 |
| Metoprolol | 1,81 | 3,43 | 1,89 |
| Pindolol | 0,86 | 1,57 | 1,83 |

Die (R,R)- bzw. gegebenenfalls (S,S)-O,O-Diacyl-, Dialkyl- sowie Dibenzylweinsäuren bzw. deren Anhydride, welche in den verschiedenen Beispielen Verwendung finden, wurden gemäß bekannter Literaturverfahren hergestellt.

Beispiel 18

Reversed-Phase HPLC Trennung der in der Tabelle VII angegebenen (R,S)-Alkanolamine als deren (R,R)-O,O-Diacetyl- bzw. (R,R)-O,O-Dibenzoylweinsäuremonoester

Die Umsetzung der (R,S)-Alkanolamine erfolgt mit den entsprechenden Weinsäureahydriden gemäß dem Beispiel 5.

Die chromatographischen Bedingungen sind:

Säule wie Beispiel 5;

Mobile Phase 1: 0,1 M $H_3PO_4$, mit $NH_3$ auf pH 3,7 eingestellt — MeOH/50—50;

Mobile Phase 2: 0,1 M $H_3PO_4$, mit $NH_3$ auf pH 3,7 eingestellt — MeOH/30—70.

Der Gesamt-pH der mobilen Phase beträgt 4,7 bzw. wird auf diesen Wert eingestellt.

TABELLE VII

| Ester von (R)-bzw. (S)-Derivat | | (R,R)-O,O-Dibenzoyl $k_R'$ | $k_S'$ | α | MP | (R,R)-O,O-Diacetyl Derivat $k_R'$ | $k_S'$ | α | MP |
|---|---|---|---|---|---|---|---|---|---|
| 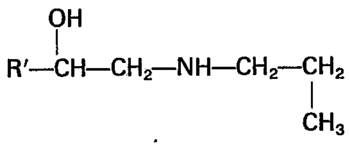 | I | 0,82 | 2,27 | 2,77 | 2 | 0,76 | 1,71 | 2,25 | 1 |
| 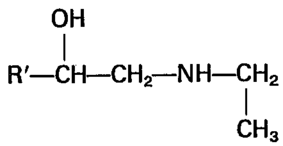 | II | 0,95 | 2,18 | 2,29 | 2 | 0,59 | 1,28 | 2,18 | 1 |
| 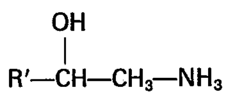 | III | 3,67 | 6,67 | 1,82 | 2 | 0,59 | 0,77 | 1,31 | 1 |
| 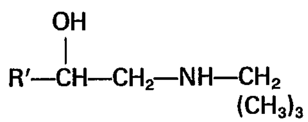 | IV | 0,95 | 2,59 | 2,73 | 2 | 1,3 | 2,45 | 1,88 | 1 |
| 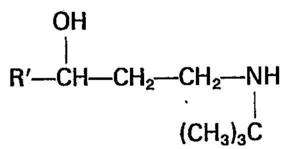 | V | 0,5 | 0,91 | 1,82 | 2 | 0,8 | 0,95 | 1,19 | 1 |
| 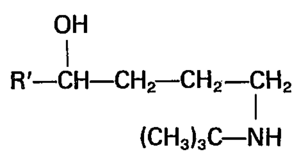 | VI | 0,82 | 1,22 | 1,49 | 2 | 1,0 | 1,28 | 1,28 | 1 |

R′= 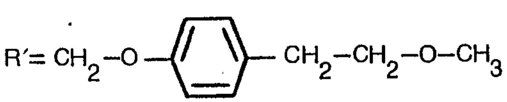

Beispiel 19

Reversed-Phase HPLC Trennung der diastereomeren (R,R)-O,O-Diarylcarbamoylweinsäuremonoester von untenstehenden (R,S)-Alkanolaminen

Die Umsetzung der (R,S)-Alkanolamine mit a) (R,R)-O,O-Di-(N-phenylcarbamoyl)-weinsäureanhydride und b) (R,R)-O,O-Di-(1-naphthyl-carbamonyl)weinsäureanhydrid wird gemäß dem Beispiel 5, jedoch mit THF als Lösungsmittel, durchgeführt.

Chromatographisches Trennsystem:

Säule wie in Biepiel 5;

Mobile Phase: 01, M Phosphorsäure mit $NH_3$ auf pH 3,6 eingestellt — MeOH/30—70.

| Ester von (R) bzw. (S) | Reagenz a) | | | Reagenz b) | | |
|---|---|---|---|---|---|---|
| | $k'_R$ | $k'_S$ | $\alpha = \frac{k'_S}{k'_R}$ | $k'_R$ | $k'_S$ | $\alpha = \frac{K'_S}{k'_R}$ |
| Propranolol | 3,39 | 5,17 | 1,51 | | | |
| 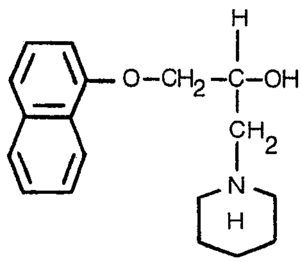 | 2,28 | 3,67 | 1,61 | 7,22 | 9,56 | 1,32 |

Die (R,R)-O,O-Di-(N-phenylcarbamoyl)-weinsäure kann wie folgt hergestellt werden:

10 g (R,R)-Weinsäurebenzylester werden in 50 ml Pyridin gelöst, auf 5°C abgekühlt und 10 g Phenylisocyanat werden zugegeben. nach 24 h bei Raumtemperatur wird auf Eiswasser gegossen. Der ölige Ruckstand wird mit Wasser gewaschen, in Dichlormethan aufgenommen un vom Ungelösten abegtrennt. Nach dem Trocknen mit Natriumsulfat und Einrotieren wird aus Ethanol umkristallisiert (14 g farblose Kristalle, Schmp. 145°C).

5 g (R,R)-O,O-Di-(N-phenylcarbamoyl)weinsäuredibenzylester werden in 50 ml Essigester gelöst und mit Pd/C 10% bei Normaldruck hydriert. Es entsteht quantitativ (R,R)-O,O-Di-(N-phenylcarbamoyl)weinsäure, Fp 200°C, Zers.

$[\alpha]^{23}_{546} = -88,6$ (c = 1,0, Methanol).

Das entsprechende Anhydrid wird in situ bei der Umsetzung mit den Alkanolaminen mit der equimolaren Menge Dicyclohexylcarbodiimid in THF hergestellt. Vollkommen analog wird die (R,R)-O,O-Di-(N-naphthylcarbamboyl)weinsäure hergestellt. Schmp. 185°C, Zer.

$[\alpha]^{23}_{546} = 29,5$ (c = 1,0, Methanol).

Tabelle I

$R_3$, $R_4$, $R_5$ = H; n = 0

| $R_2$ | $R_6$ | Name des Alkanolamins |
|---|---|---|
| 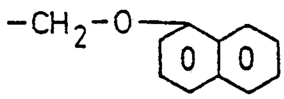 | Isopropyl | Propranolol |
| 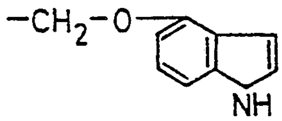 | Isopropyl | Pindolol |
| 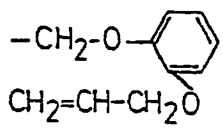 | Isopropyl | Oxprenolol |
| 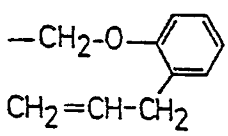 | Isopropyl | Alprenolol |
| 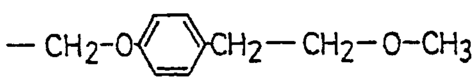 | Isopropyl | Metoprolol |
| 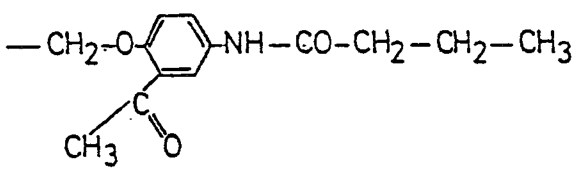 | Isopropyl | Acebutolol |
| 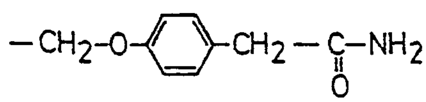 | Isopropyl | Atenolol |
| 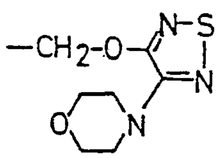 | t-Butyl | Timolol |
| 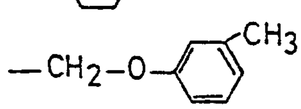 | Isopropyl | Toliprolol |
| 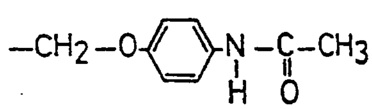 | Isopropyl | Practolol |
| 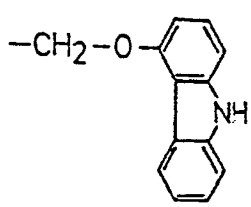 | Isopropyl | Carazolol |
| 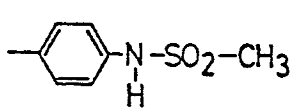 | Isopropyl | Sotalol |

Tabelle I (Fortsetzung)

$R_3$, $R_4$, $R_5$ = H; n = 0

| $R_2$ | $R_6$ | Name des Alkanolamins |
|---|---|---|
| 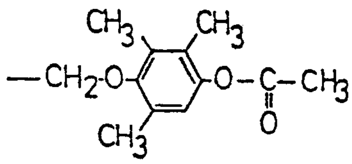 | Isopropyl | Methypranolol |
| 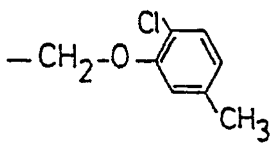 | t-Butyl | Bupranolol |
| 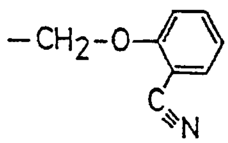 | t-Butyl | Bunitrolol |
| 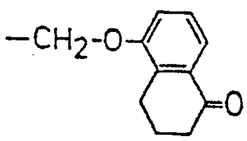 | t-Butyl | Bunolol |
| 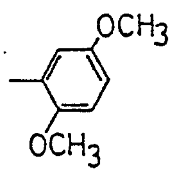 | $-CO-CH_2NH_2$ | Midodrin |
| 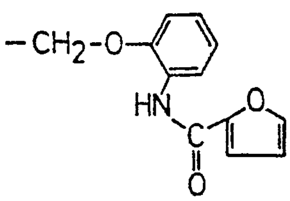 | t-Butyl | Ancarolol |
| 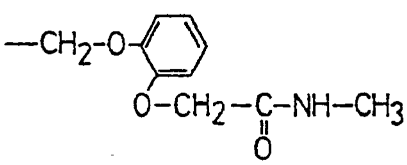 | t-Butyl | Cetamolol |
| 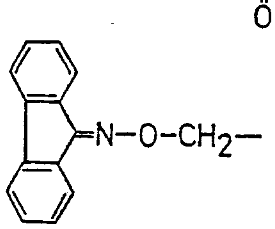 | t-Butyl | |
| 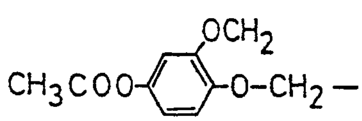 | t-Butyl | Vanilol |
| 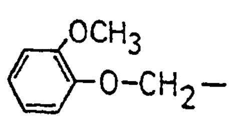 | Isopropyl | Moprolol |

Tabelle I (Fortsetzung)

$R_3, R_4, R_5 = H; n = 0$

| $R_2$ | $R_6$ | Name des Alkanolamins |
|---|---|---|
| 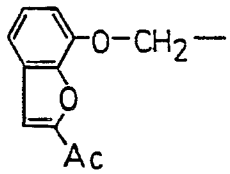 | Isopropyl | Befunolol |
| 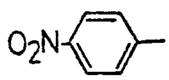 | Isopropyl | Nifenalol |
| 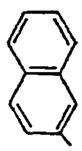 | Isopropyl | Pronethalol |
| 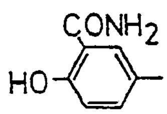 | 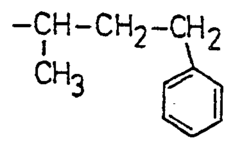 | Labetalol |
| 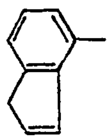 | Isopropyl | Indenol |
| 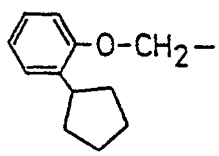 | t-Butyl | Penbutolol |
| 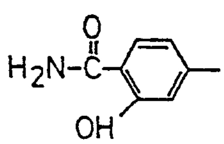 | 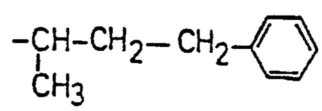 | |
| 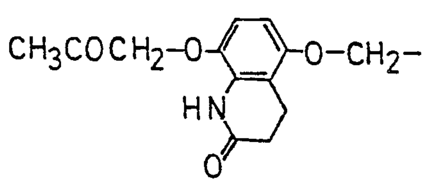 | 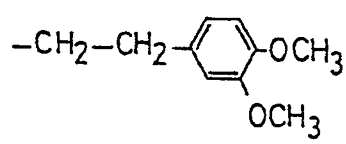 | Bometolol |
| 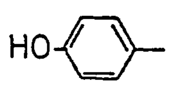 | n-Butyl oder Methyl | Bamethan |
| 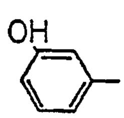 | Ethyl | |
| $Cl-CH_2-$ | Isopropyl | |
| $Tos-O-CH_2-$ | Isopropyl | |

Tabelle I (Fortsetzung)

$R_3$, $R_4$, $R_5$ = H; n = 0

| $R_2$ | $R_6$ | Name des Alkanolamins |
|---|---|---|
| 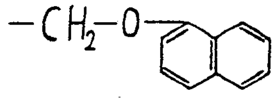 | Wasserstoff | Norpropranolol |
| 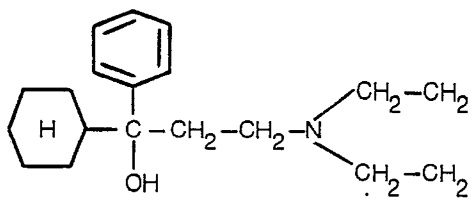 | | Procyclidin |
| 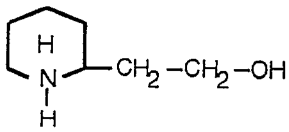 | | |
| 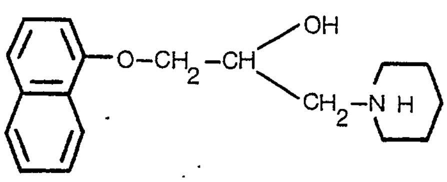 | | |

## Patentansprüche

1. Weinsäuremonoester von optisch aktiven, substituierten Alkanolaminen der allgemeinen Formel

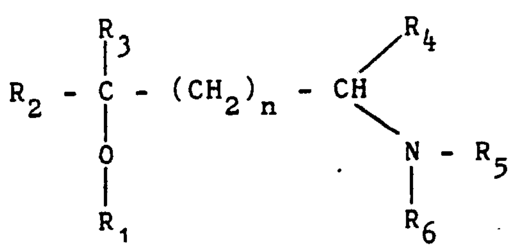

sowie deren Salze, in der

n null bis vier bedeutet,

$R_1$ (R,R)- oder (S,S)-tartryl bedeutet, in dem der Wasserstoff der Hydroxylgruppen durch einen Acyl-, alkyl-, Aralkyl-, N-Arylcarbamoyl- oder Alkenylrest, die jeweils substituiert sein können, substituiert ist,

$R_2$ Alkyl, Aralkyl, Aryl, Methylenfluorenonoximether, die Gruppe $R_7$-oxyalkyl, in der $R_7$ Aryl oder ein heterocyclischer Rest ist, wobei die Reste jeweils substiuiert sein können, oder Wasserstoff bedeutet,

$R_3$ Aryl, Alkyl, Cycloalkyl, wobei die Reste jeweils substituiert sein können, oder Wasserstoff bedeutet,

$R_4$ Alkyl, Aryl, wobei die Reste substituiert sein können, oder Wasserstoff bedeutet,

$R_5$ und $R_6$, die gleich oder verschieden sein können, geradkettiges, verzweigtes oder cyclisches Alkyl, einen 5- oder 6-gliedrigen stickstoffhaltigen Heterocyclus, Aralkyl oder Aminoalkylcarbonyl, wobei die Reste jeweils substituiert sein können, oder Wasserstoff bedeutet, und in der die Reste $R_2$ und $R_5$ und/oder $R_5$ und $R_6$ ringförmig miteinander verknüpft sein können, wobei die Weinsäuremonoester des Celiprolols ausgenommen sind.

2. Weinsäuremonoester nach Anspruch 1, dadurch gekennzeichnet, daß im Rest $R_1$ der Acylrest die Acetyl-, ggf. substituierte Phenylacetyl-, Toluoyl-, ggf. substituierte Benzoyl- oder Trichloracetylgruppe ist, der Alkylrest 1 bis 5 C-Atome enthält, der Aralkylrest Benzyl ist, und der Alkenylrest 2 bis 5 C-Atome enthält, wobei die Reste jeweils substituiert sein können.

3. Weinsäuremonoester nach Anspruch 2, dadurch gekennzeichnet, daß der Acylrest die Acetyl-, Benzoyl- oder Toluoylgruppe, der Alkylrest die Methyl-, Ethyl- oder tert. Butylgruppe, der Alkenylrest die Vinyl- oder 2,2-Dichlorvinylgruppe und der N-Arylcarbamoylrest die N-Phenylcarbamoyl- oder N-Naphthylcarbamoylgruppe bedeutet.

4. Weinsäuremonoester nach einem der Ansprüche 1 bis 3, dadurch gekennzeichnet, daß

n null, eins oder zwei ist,

$R_2$ Alkyl mit 1 bis 3 C-Atomen, Aralkyl mit 7 bis 10 C-Atomen, Aryl, Methylenfluorenonoximether, die Gruppe $R_7$-oxymethyl, wobei die Reste jeweils substituiert sein können, oder Wasserstoff bedeutet,

$R_3$ Phenyl, Cyclohexyl oder Wasserstoff,

$R_4$ Alkyl mit 1 bis 3 C-Atomen oder Wasserstoff,

$R_5$ und $R_6$ Wasserstoff, ein verzweigter oder geradkettiger Alkylrest mit 1 bis 4 C-Atomen, ein cyclischer Alkylrest mit 4 bis 6 C-Atomen, ein Aminoalkylcarbonylrest oder ein Phenylalkylrest mit 7 bis 12 C-Atomen bedeuten.

5. Weinsäuremonoester nach Anspruch 4, dadurch gekennzeichnet, daß

$R_2$ als Alkylgruppe mit Chlor oder dem Tosyloxyrest substituiert ist, und als Arylgruppe Phenyl, Indenyl oder Naphthyl ist, die jeweils substituiert sein können, und

$R_7$ als Arylgruppe Phenyl, Naphthyl, Indenyl oder ein Benzocycloalkylrest, die ggf. substituiert sein können, und als heterocyclischer Rest ein fünf- oder sechsgliedriger Heterocyclus oder ein Benzoheterocyclus ist.

6. Weinsäuremonoester nach Anspruch 5, dadurch gekennzeichnet, daß

$R_2$ als Arylgruppe der Phenylrest ist, der ggf. substituiert sein kann, und

$R_7$ als Arylgruppe ein substituierter Phenyl-, Naphthyl- oder Indeylrest und als heterocyclischer Rest der Indolyl-, Thiodiazolyl-, Carbazolyl-, Benzofuranyl- oder Dihydrocarbostyrinylrest ist, die substiuiert sein können.

7. Weinsäuremonoester nach einem der Ansprüche 1 bis 6, dadurch gekennzeichnet, daß $R_2$ und $R_5$ und/oder $R_5$ und $R_6$ durch 2 bis 5 Kohlenstoffatome miteinander verknüpft sind.

8. Weinsäuremonoester nach einem der Ansprüche 1 bis 6, dadurch gekennzeichnet, daß $R_4$ und $R_5$ Wasserstoff, $R_6$ die Ethyl-, Propyl-, Isopropyl-, tert. Butyl- oder Aminomethylcarbonylgruppe und n null ist.

9. Weinsäuremonoester und deren Salze nach einem der Ansprüche 1 bis 8, dadurch gekennzeichnet, daß sie in optisch reiner Form vorliegen, wobei das Celiprolol ausgenommen ist.

10. Verfahren zur Herstellung von Weinsäuremonoestern nach einem der Ansprüche 1 bis 8, wobei von einem Gemisch der optischen Antipoden eines Alkanolamins der allgemeinen Formel

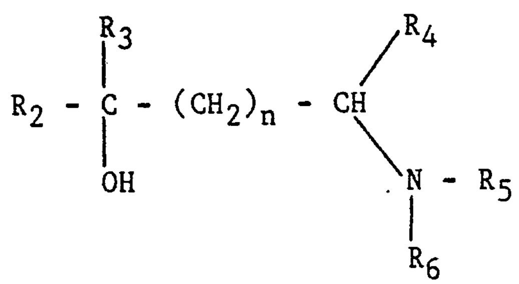

(II)

ausgegangen wird, in der $R_2$ bis $R_6$ und n die angegebenen Bedeutungen haben, wobei Celiprolol ausgenommen ist, dadurch gekennzeichnet, daß die Aminogruppe des Alkanolamins, sofern sie eine primäre oder sekundäre Aminogruppe ist, ggf. geschützt wird, und das Alkanolamin in der Schmelze oder in einem aprotischen Lösungsmittel mit einer (R,R,)- bzw. (S,S)-Weinsäure, in der der Wasserstoff der Hydroxylgruppen durch einen Acyl-, Alkyl-, Aralkyl-, N-Arylcarbamoyl- oder Alkenylrest, die jeweils substiuiert sein können, substituiert ist, in Gegenwart eines Kondensationsmittels oder mit dm Anhydrid der disubstituierten Weinsäuren umgesetzt wird, und, sofern die Aminogruppe geschützt wurde, die Schutzgruppe wieder entfernt wird.

11. Verfahren nach Anspruch 10, dadurch gekennzeichnet, daß die primäre oder sekundäre Aminogruppe durch Umsetzung mit einer anorganischen oder organischen Säure geschützt wird, die mit der Aminogruppe eine Ionenpaarbindung eingeht, die in dem aprotischen Lösungsmittel oder in der Schmelze nur schwach dissoziiert.

12. Verfahren nach Anspruch 11, dadurch gekennzeichnet, daß als organische Säure Sulfonsäuren oder Halogencarbonsäuren und als anorganische Säuren Halogenwasserstoffsäuren verwendet werden.

13. Verfahren nach Anspruch 12, dadurch gekennzeichnet, daß als organische Säure Toluolsulfonsäuren, Trichloressigsäure oder Trifluoressigsäure und als anorganische Säure Salzsäure verwendet werden.

14. Verfahren nach einem der Ansprüche 10 bis 13, dadurch gekennzeichnet, daß die gebildeten Weinsäuremonoester der Alkanolamine aus wäßrigem Medium, insbesondere im pH-Bereich zwischen 0 und 9, kristallisiert oder mittels organischer Lösungsmittel, insbesondere Dichlormethan oder Dichlorethan, extrahiert werden.

15. Verfahren nach einem der Ansprüche 10 bis 14, dadurch gekennzeichnet, daß als aprotisches Lösungsmittel Dichlormethan, 1,2-Dichlorethan, Aceton, Acetonitrile, Toluol, Dimethylformamid, Dimethylsulfoxid, Tetrahydrofuran, Dioxan, Ethylenglykoldimethylether oder N-Methylpyrrolidon-2-verwendet werden.

16. Verfahren nach einem der Ansprüche 10 bis 15, dadurch gekennzeichnet, daß die Veresterung in einem aprotischen Lösungsmittel bei Temperaturen zwischen Raumptemperatur und 90°C durchgeführt wird.

17. Verfahren zur Herstellung der optisch reinen Weinsäuremonoester nach Anspruch 9, dadurch gekennzeichnet, daß das nach einem der Ansprüche 10 bis 16 erhaltene Isomerengemisch der diastereomeren Weinsäuremonoester durch Extraktion mit organischen Lösungsmitteln, in denen die Ester nicht oder nur langsam solvolysierbar sind, in die optisch reinen Isomeren aufgetrennt wird.

18. Verfahren nach Anspruch 17, dadurch gekennzeichnet, daß als organische Lösungsmittel Aceton, Isopropanol, Dichlormethan, Dichlorethan, Chloroform, Essigester, Dioxan, Tetrahydrofuran, verschiedene Ether oder Cyclohexan-Toluol verwendet werden.

19. Verfahren zur Herstellung der optisch reinen Weinsäuremonoester nach Anspruch 9, dadurch gekennzeichnet, daß das nach einem der Ansprüche 10 bis 16 erhaltene Isomerengemisch der diastereomeren Weinsäuremonoester durch pH kontrollierte wäßrige Extraktion aufgrund unterschiedlicher Löslichkeiten in die optisch reinen Isomeren aufgetrennt wird.

20. Verfahren nach Anspruch 19, dadurch gekennzeichnet, daß in einem pH-Bereich zwischen 1 und 8 gearbeitet wird, und organische oder anorganische wäßrige Pufferlösungen verwendet werden.

21. Verfahren zur Herstellung der optisch reinen Weinsäuremonoester nach Anspruch 9, dadurch gekennzeichnet, daß das nach einem der Ansprüche 10 bis 16 erhaltene Isomerengemisch der diastereomeren Weinsäuremonoester durch chromatographische Trennverfahren, insbesondere Adsorptionschromatographie, wie Kieselgel- oder Aluminiumoxidsäulen oder Dünnschicht-Chromatographie oder Verteilungschromatographie, wie Reversed-Phase Säulenchromatographie, in die optisch reinen Isomeren aufgetrennt wird.

22. Verfahren zur Herstellung der optisch reinen Weinsäuremonoester nach Anspruch 9, dadurch gekennzeichnet, daß das nach einem der Ansprüche 10 bis 16 erhaltene Isomerengemisch der diasteromeren Weinsäuremonoester durch fraktionierte Kristallisation unter Bedingungen, bei denen die Ester nicht oder nur langsam solvolysieren, in die optisch reinen Isomeren aufgretennt wird.

23. Verwendung der Weinsäuremonoester nach einem der Ansprüche 1 bis 9 zur Herstellung optisch reiner Akanolamine der allgemeinen Formel

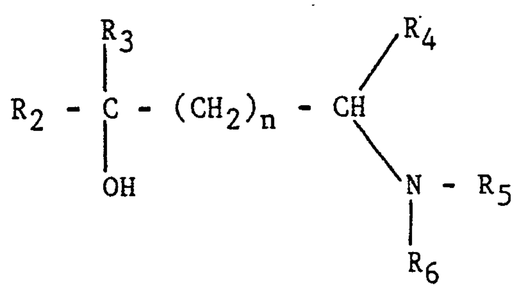

(II)

in der $R_2$ bis $R_6$ und n die angegebenen Bedeutungen haben, wobei das Celiprolol ausgenommen ist, indem die getrennten optisch reinen Isomeren der Alkanolaminweinsäuremonoester unter saure oder basischen Bedingungen oder durch Umesterung solvolytisch gespalten werden.

24. Verwendung der Weinsäuremonoester nach einem der Ansprüche 1 bis 9 zur Herstellung optisch reiner Alkanolamine der allgemeinen Formel

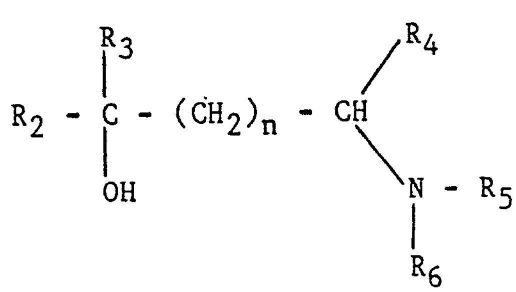

(II)

in der $R_2$ bis $R_6$ und n die angegebenen Bedeutungen haben, wobei das Celiprolol ausgenommen ist, indem das Gemisch der diastereomeren Alkanolaminweinsäuremonoester einer stereospezifischen pH kontrollierten Hydrolyse oder eine stereospezifischen enzymatischen Hydrolyse unterworfen wird.

**Revendications**

1. Monoester d'acide tartrique d'aminoalcools substitués optiquement actifs, de formule générale

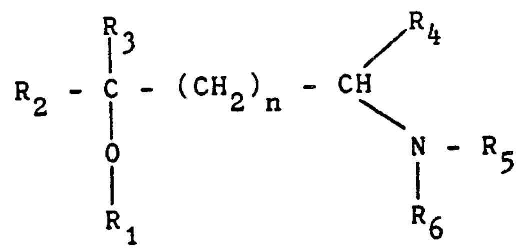

ainsi que de leurs sels, dans laquelle

n représente un chiffre allant de 0 à 4,

$R_1$ représente un radical (R,R)-tartryle ou (S,S,)-tartryle dans lequel l'hydrogène des groupes hydroxyle est substitué par un radical acyle alkyle, aralkyle, N-arylcarbamoyle ou alkényle, qui peuvent être respectivement substitués,

$R_2$ représente un alkyle, un aralkyle, un aryle, un éther de méthylène-fluorène-onoxime, le groupe $R_7$-oxyalkyle dans lequel $R_7$ est un aryle ou un radical hétérocyclique, les radicaux pouvant être respectivement substitués, ou l'hydrogène,

$R_3$ représente un aryle, un alkyle, un cycloalkyle, les radicaux pouvant être respectivement substitués, ou l'hydrogène,

$R_4$ représente un alkyle, un aryle, les radicaux pouvant être substitués, ou l'hydrogène,

$R_5$ et $R_6$, qui peuvent être identiques ou différents, représentent un alkyle linéaire, ramifié ou cyclique, un hétérocycle azoté à 5 ou 6 chaînons, un aralkyle ou un aminoalkylcarbonyle, les radicaux pouvant être respectivement subtitués, ou l'hydrogène, et dans lesquels les radicaux $R_2$ et $R_5$ et/ou $R_5$ et $R_6$ peuvent être reliés entre eux sous forme d'anneau,

dans lesquels les monoesters d'acide tartrique du céliprolol sont exclus.

2. Monoesters d'acide tartrique selon la revendication 1, caractérisés en ce que, dans le radical $R_1$, le radical acyle est le groupe acétyle, le cas échéant le groupe phénylacétyle substitué, le groupe toluoyle, le cas échéant le groupe benzoyle substitué ou le groupe trichloro-acétyle, le radical alkyle contient de 1 à 5 atomes de carbone, le radical aralkyle est un benzyle et le radical akényle contient de 2 à 5 atomes de carbone, les radicaux pouvant être respectivement substitués.

3. Monesters d'acide tartrique selon la revendication 2, caractérisés en ce que le radical acyle représente le groupe acétyle, benzoyle ou toluoyle, le radical alkyle représente le groupe méthyle, éthyle, ou tert-butyle, le radical alkényle représente le groupe vinyle ou 2,2-dichlorovinyle et le radical N-arylcarbamoyle représente le groupe N-phénylcarbamoyle ou N-naphthylcarbamoyle.

4. Monoester d'acide tartrique selon l'une des revendications 1 à 3, caractérisés en ce que

n est égal à 0, 1 ou 2,

$R_2$ représente un alkyle contenant de 1 à 3 atomes de carbone, un aralkyle contenant de 7 à 10 atomes de carbone, un aryle, un éther de méthylène-fluorène-onoxime, le groupe $R_7$-oxyméthyle, les radicaux pouvant être respectivement substitués, ou l'hydrogène.

$R_3$ représente un phényle, un cyclohexyle ou l'hydrogène,

$R_4$ représente un alkyle contenant de 1 à 3 atomes de carbone, ou l'hydrogène,

$R_5$ et $R_6$ représentent l'hydrogène, un radical alkyle ramifié ou linéaire contenant de 1 à 4 atomes de carbone, un radical alkyle cyclique contenant de 4 à 6 atomes de carbone, un radical aminoalkylcarbonyle ou un radical phénylalkyle contenant de 7 à 12 atomes de carbone.

5. Monoesters d'acide tartrique selon la revendication 4, caractérisés en ce que

$R_2$ en tant que groupe alkyle, est substitué par du chlore ou par le radical tosyloxy et, en tant que groupe aryle, représente un phényle, un indényle ou un naphthyle qui peuvent être respectivement substitués,

$R_7$ en tant que groupe aryle, représente un phényle, un naphthyle, un indényle ou un radical benzo-cycloalkyle, qui peuvent être éventuellement substitués et, en tant que radical hétérocyclique, représente un hétérocycle à 5 ou 6 chaînons ou un benzohétérocycle.

6. Monoesters d'acide tartrique selon la revendication 5, caractérisés en ce que

$R_2$ en tant que groupe aryle, est le radical phényle qui, le cas échéant, peut être substitué et,

$R_7$ en tant que groupe aryle, est un radical substitué phényle, naphthyle ou indényle et, en tant que radical hétérocyclique, le radical indolyle, thiodiazolyle, carbazolyle, benzofuranyle ou dihydro-carbostyrinyle, qui peuvent être substitués.

7. Monoesters d'acide tartrique selon l'une des revendications 1 à 6, caractérisés en ce que $R_2$ et $R_5$ et/ou $R_5$ et $R_6$ sont reliés entre eux par 2 à 5 atomes de carbone.

8. Monoester d'acide tartrique selon l'une des revendications 1 à 7, caractérisés en ce que $R_4$ et $R_5$ sont chacun de l'hydrogène, $R_6$ est le groupe éthyle, propyle, isopropyle, tert-butyle ou aminoéthylcarbonyle et n est égal à zéro.

9. Monoesters d'acide tartrique et leurs sels selon l'une des revendications 1 à 8, caractérisés en ce qu'ils se présentent sous une forme optiquement pure, dans lesquels le céliprolol est exclu.

10. Procédé de préparation de monoesters d'acide tartrique selon l'une des revendications 1 à 8, dans lequel on part d'un mélange des inverses optiques d'un aminoalcool de formule générale

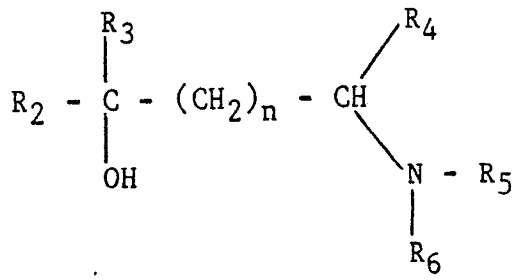

(II)

dans laquelle les indices $R_2$ à $R_6$ et n ont les significations précitées, le céliprolol étant exclu, caractérisé en ce que le groupe amino de l'aminoalcool, dans la mesure où il s'agit d'un groupe amino primaire ou secondaire, est protégé le cas échéant, et on fait réagir l'aminoalcool dans la masse fondue ou dans un solvant aprotique, en présence d'un agent de condensation ou de l'anhydride des acides tartriques disubstitués, avec un (R,R)- ou (S,S)-acide tartrique dans lequel l'hydrogène des groupes hydroxyle est substitué par un radical acyle, alkyle, aralkyle, N-arylcarbamoyle ou alkényle qui peuvent être respectivement substitués, et, dans la mesure où le groupe amino avait été protégé, on élimine de nouveau le groupe protecteur.

11. Procédé selon la revendication 10, caractérisé en ce que le groupe amino primaire ou secondaire est protégé par réaction avec un acide inorganique ou organique qui forme une liaison de paires d'ions avec le groupe amino, laquelle liaison ne se dissocie que très faiblement dans le solvant aprotique ou dans la masse fondue.

12. Procédé selon la revendication 11, caractérisé en ce qu'on utilise comme acides organiques des acides sulfoniques ou des hydracides halogénés et, comme acides inorganiques, des haloacides.

13. Procédé selon la revendication 12, caractérisé en ce qu'on utilise comme acides organiques des acides toluolsulfoniques, de l'acide trichloro-acétique ou de l'acide trifluoro-acétique et, comme acide inorganique, de l'acide chlorhydrique.

14. Procédé selon l'une des revendications 10 à 13, caractérisé en ce que les monoesters d'acide tartrique formés des aminoalcools sont cristallisés à partir d'un milieu aqueux, en particulier avec un pH compris entre 0 et 9, ou sont extraits au moyen de solvants organiques, en particulier du dichlorométhane ou du dichloro-éthane.

15. Procédé selon l'une des revendications 10 à 14, caractérisé en ce qu'on utilise comme solvant aprotique du dichlorométhane, du 1,2-dichloro-éthane, de l'acétone, de l'acétonitrile, du toluène, du diméthylformamide, du diméthylsulfoxyde, du tétrahydrofurane, du dioxanne, un diméthylether éthylène-glycol ou de la N-méthyl-pyrrolidone-2.

16. Procédé selon l'une des revendications 10 à 15, caractérisé en ce que l'estérification est réalisée dans un solvant aprotique à une température comprise entre la température ambiante et 90°C.

17. Procédé de préparation des monoesters d'acide tartrique optiquement purs selon la revendication 9, caractérisé en ce que le mélange isomérique des monoesters diastéréomères d'acide tartrique, mélange obtenu selon l'une des revendications 10 à 16, est séparé en isomères optiquement purs par extraction au moyens de solvants organiques dans lesquels les esters ne sont pas solvolysables, ou ne le sont que lentement.

18. Procédé selon la revendication 17, caractérisé en ce qu'on utilise comme solvants organiques de l'acétone, de l-isopropanol, du dichlorométhane, du dichloro-éthane, du chloroforme, de l'acétate d'éthyle, du dioxanne, du tétrahydrofurane, différents éthers ou du cyclohexane-toluène.

19. Procédé de préparation des monoesters d'acide tartrique optiquement purs selon la revendication 9, caractérisé en ce que le mélange isomérique des monoesters diastéréomères d'acide tartrique, mélange obtenu selon l'une des revendications 10 à 16, est séparé en isomères optiquement purs par extraction aqueuse contrôlée par pH, en fonction de solubilités différentes.

20. Procédé selon la revendication 19, caractérisé en ce qu'on travaille dans un mileu pH compris entre 1 et 8 et en ce qu'on utilise des solutions aqueuses tamponnées organiques ou inorganiques.

21. Procédé de préparation des monoesters d'acide tartrique optiquement purs selon la revendication 9, caractérisé en ce que le mélange isomérique des monoesters diastéréomères d'acide tartrique, mélange obtenu selon l'une des revendications 10 à 16, est séparé en isomères optiquement purs selon des procédés chromatographiques de séparation, notamment par chromatographie par adsorption, par exemple au moyen de colonnes à gel de silice ou à oxyde d'aluminium, ou par chromatographie en couche mince, ou par chromatographie de partage, par exemple par chromatographie sur colonnes à phases inversées.

22. Procédé de préparation des monoesters d'acide tartrique optiquement purs selon la revendication 9, caractérisé en ce que le mélange isomérique des monoesters diastéréomères d'acide tartrique, mélange

obtenu selon l'une des revendications 10 à 16, est séparé en isomères optiquement purs par cristallisation fractionnée dans des conditions selon lequelles les esters ne sont pas solvolysés ou ne le sont que lentement.

23. Utilisation des monoesters d'acide tartrique selon l'une des revendications 1 à 9 pour préparer des aminoalcools optiquement purs de formule générale

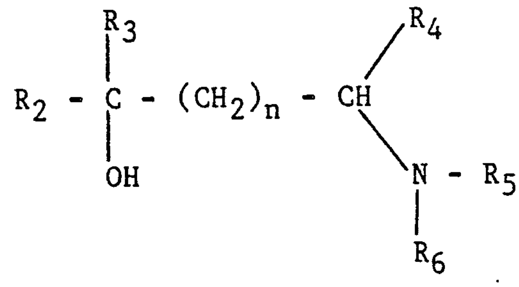

(II)

dans laquelle les indices $R_2$ à $R_6$ et n ont les significations précitées, le céliprolol étant exclu, caractérisée en ce que les isomères séparés et optiquement purs des monoesters d'acide tartrique d'aminoalcools sont fragmentés par solvolyse en milieu acide ou basique ou par transestérification.

24. Utilisation des monoesters d'acide tartrique selon l'une des revendications 1 à 9 pour préparer des aminoalcools optiquement purs de formule générale

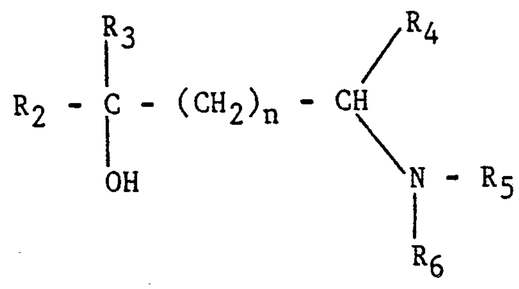

(II)

dans laquelle les indices $R_2$ à $R_6$ et n ont les significations précitées, le céliprolol étant exclu, caractérisée en ce que le mélange de monoesters diastéréomères d'acide tartrique d'aminoalcools est soumis à une hydrolyse stéréospécifique contrôlée par pH ou à une hydrolyse enzymatique stéréospécifique.

**Claims**

1. Tartaric acid monoesters of optically active, substituted alkanolamines of the general formula

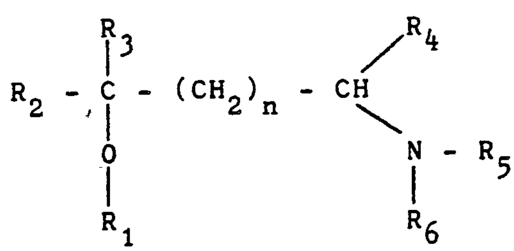

and also of their salts, wherein

n can have the value nought to four,

$R_1$ signifies (R,R) tartryl or (S,S) tartryl, in which the hydrogen of the hydroxyl groups is substituted by an acyl, alkyl, aralkyl, N-arylcarbamoyl or alkenyl radical, which can in each case be substituted,

$R_2$ signifies alkyl, aralkyl, aryl, methylene fluorenone oxime ether, the group $R_7$-oxyalkyl, in which $R_7$ is aryl or a heterocyclic radical, in which the radicals can in each case be substituted, or hydrogen,

$R_3$ signifies aryl, alkyl, cycloalkyl, in which the radicals can in each case be substituted, or hydrogen,

$R_4$ signifies alkyl, aryl, in which the radicals can in each case be substituted, or hydrogen,

$R_5$ and $R_6$, which can either be the same or different, signify straight chained, branched or cyclic alkyl, a five or six membered nitrogen containing heterocycle, aralkyl or amino alkyl carbonyl, in which the radicals can in each case be substituted, or hydrogen and in which the radicals $R_2$ and $R_5$ and/or $R_5$ and $R_6$ can be linked with one another in ring-like manner, and wherein the tartaric acid monoesters or celiprolol are excluded.

2. Tartaric acid monoesters in accordance with claim 1, characterized in that in the radical $R_1$ the acyl radical is the acetyl group, the optionally substituted phenyl acetyl group, the toluoyl group, the optionally substituted benzoyl group or the trichloroacetyl group, the alkyl radical contains 1 to 5 C-atoms, the aralkyl radical is benzyl, and the alkenyl radical contains 2 to 5 C-atoms, wherein the radicals can in each case be substituted.

3. Tartaric acid monoesters in accordance with claim 2, characterized in that the acyl radical signifies the acetyl group, the benzoyl group or the toluoyl group, the alkyl radical signifies the methyl group, the ethyl group or the tertiary butyl group, the alkenyl radical signifies the vinyl group or the 2.2 dichlorovinyl group and the N-aryl carbamoyl radical signifies the N-phenyl carbamoyl group or the N-naphthyl carbamoyl group.

4. Tartaric acid monoesters in accordance with one of the claims 1 to 3, characterized in that

n is 0, 1 or 2,

$R_2$ signifies alkyl with 1 to 3 carbon atoms, aralkyl with 7 to 10 carbon atoms, aryl, methylene fluorenone oxime ether, the group $R_7$-oxymethyl, wherein the radicals can in each case be substituted, or hydrogen,

$R_3$ signifies phenyl, cyclohexyl or hydrogen,

$R_4$ signifies alkyl with 1 to 3 C-atoms or hydrogen,

$R_5$ and $R_6$ signify hydrogen, a branched or straight chained alkyl radical with 1 to 4 carbon atoms, a cyclical alkyl radical with 4 to 6 C-atoms, an amino alkyl carbonyl radical or a phenyl alkyl radical with 7 to 12 C-atoms.

5. Tartaric acid monoesters in accordance with claim 4, characterized in that

$R_2$ as an alkyl group is substituted with chlorine or the tosyloxy radical, and as an aryl group is phenyl, indenyl or naphthyl, which can in each case be substituted, and

$R_7$ as an aryl group is phenyl, naphthyl, indenyl or a benzocycloalkyl radical and these can optionally be substituted, and, as a heterocyclic radical, is a five or six member heterocycle or a benzoheterocycle.

6. Tartaric acid monoesters in accordance with claim 5, characterized in that

$R_2$ as an aryl group is the phenyl radical, which can optionally be substituted, and

$R_7$ as an aryl group is a substituted phenyl, naphthyl or indenyl radical and as a heterocyclical radical is the indolyl, thiodiazolyl, benzofuranyl or dihydrocarbostyrinyl radical, wherein these radicals can be substituted.

7. Tartaric acid monoesters in accordance with one of the claims 1 to 6, characterized in that $R_2$ and $R_5$ and/or $R_5$ and $R_6$ are linked with one another by 2 to 5 carbon atoms.

8. Tartaric acid monoesters in accordance with one of the claims 1 to 6, characterized in that $R_4$ and $R_5$ are hydrogen, $R_6$ is the ethyl, propyl, isopropyl, tertiary butyl or amino methyl carbonyl group and n is zero.

9. Tartaric acid monoesters and their salts in accordance with anyone of the claims 1 to 8, characterized in that they are present in optically pure form, with celiprolol being excluded.

10. A method of synthesizing tartaric acid monoesters in accordance with one of the claims 1 to 8, starting from a mixture of the optically isomeric forms of an alkanolamine of the general formula

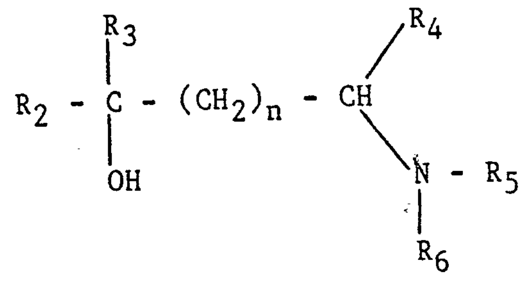

(II)

in which $R_2$ to $R_6$ and n have the meanings given, with celiprolol being excluded, characterized in that the amino group of the alkanolamines, to the extent that it is a primary or secondary amino group, is optionally protected, and the alkanolamine is reacted in the melt or in an aprotic solvent with an (R,R)- or (S,S)-tartaric acid in which the hydrogen of the hydroxyl groups is substituted by an acyl, alkyl, aralkyl, N-aryl carbamoyl or alkenyl radical, which can in each case be substituted, in the presence of a condensation agent or with the anhydride of the disubstituted tartaric acids and, in so far as the amino group is protected, the protective group is removed again.

11. A method in accordance with claim 10, characterized in that the primary or secondary amino group is protected by reaction with an inorganic or organic acid, which forms an ion pair bond with the amino group which only weakly dissociates in the aprotic solvent or in the melt.

12. A method in accordance with claim 11, characterized in that acids or halogenated carboxylic acids are used as organic acids and hydrohalogenic acids are used as inorganic acids.

13. A method in accordance with claim 12, characterized in that toluene sulfonic acids, trichloro acetic acids or trifluoro acetic acids are used as organic acids and hydrochloric acid is used as the inorganic acid.

14. Procédé selon l'une des revendications 10 à 13, caractérisé en ce que les monoesters d'acide monoesters of the alkanolamines that are formed are crystallized out of the aqueous medium, in particular in the pH range between 0 and 9, or are extracted by means of organic solvents, in particular dichloromethane or dichloroethane.

15. A method in accordance with one of the claims 10 to 14, characterized in that dichloromethane, 1.2-dichloroethane, acetone, acetonitrile, toluene, dimethylformamide, dimethylsulfoxide, tetrahydrofuran, dioxan, ethylene glycol dimethylether or N-methyl-pyrrolidone-2 are used as an aprotic solvent.

16. A method in accordance with one of the claims 10 to 15, characterized in that the esterification is carried out in an aprotic solvent at temperatures between room temperature and 90°C.

17. A method of manufacturing the optically pure tartaric acid monoesters of claim 9, characterized in that the isomer mixture of the diastereoisomeric tartaric acid monoesters is separated into the optically pure isomers by extraction with organic solvents in which the esters are not or are only slowly solvolysable.

18. A method in accordance with claim 17, characterized in that acetone, isopropanol, dichloromethane, dichloroethane, chloroform, acetic ester, dioxan, tetrahydrofuran or cyclohexane/toluene are used as the organic solvent.

19. A method of manufacturing the optically pure tartaric acid monoesters of claim 9, characterized in that the isomer mixture of the diastereoisomeric tartaric acid monoesters that is obtained in accordance with one of the claims 10 to 16 is separated into the optically pure isomers by pH controlled aqueous extraction as a result of different solubilities.

20. A method in accordance with claim 19, characterized in that the separation is carried out in a pH range between 1 and 8 and organic or inorganic aqueous buffer solutions are used.

21. A method of manufacturing the optically pure tartaric acid monoesters of claim 9, characterized in that the isomer mixture of the diasteroisometric tartaric acid monoesters that is obtained in accordance with one of the claims 10 to 16 is separated into the optically pure isomers by chromatographic separation methods, in particular adsorption chromatography, such as silica gel columns or aluminium oxide columns or thin layer chromatography, or partition chromatography such as reversed phase column chromatography.

22. A method of manufacturing the optically pure tartaric acid monoesters of claim 9, characterized in that the isomer mixture of the diastereoisomeric tartaric acid monoesters that is obtained in accordance with one of the claims 10 to 16 is separated into the optically pure isomers by fractionated crystallization under conditions in which the esters are not or are only slowly solvolysable.

23. The use of tartaric acid monoesters in accordance with one of the claims 1 to 9, for the manufacture of optically pure alkanolamines of the general formula

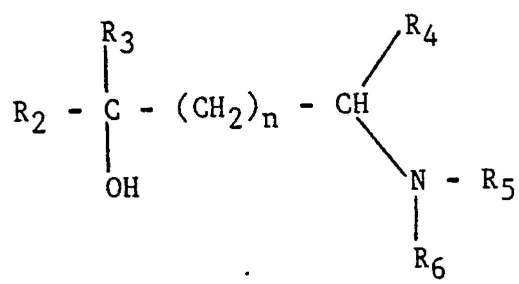

(II)

in which $R_2$ to $R_6$ and n have the meanings given, with celiprolol being excluded, in which the separated optically pure isomers of the alkanolamine tartaric acid monoesters are cleaved by solvolysis under acidic or basic conditions or by transesterification.

24. The use of tartaric acid monoesters in accordance with one of the claims 1 to 9 for the manufacture of optically pure alkanolamines of the general formula

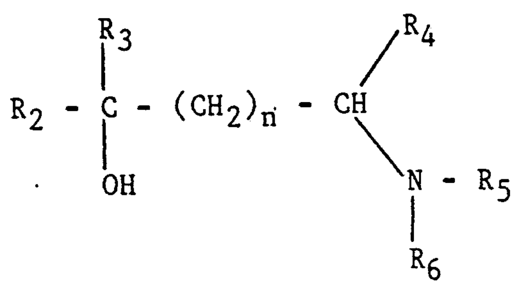

(II)

in which $R_2$ to $R_6$ and n have the meanings given, with celiprolol being excluded, in which the mixture of the diastereoisomeric alkanolamine tartaric acid monoesters is subjected or to a stereospecific pH controlled hydrolysis or to a stereospecific enzymatic hydrolysis.